# EUROPEAN PATENT APPLICATION

(11) **EP 4 755 384 A1**
(43) Date of publication of application: **10.06.2026**
(21) Application number: 24862075.9
(22) Date of filing: 06.09.2024
(51) Int. Cl.: A61K 31/506, C07D 401/04, A61P 17/14, A61P 17/10, A61P 5/28

(54) **PHARMACEUTICAL COMPOSITION CONTAINING ANDROGEN RECEPTOR INHIBITOR/ANTAGONIST, METHOD AND USE**

(30) Priority: 08.09.2023 CN 202311157854; 06.02.2024 CN 202410171642
(71) Applicant: Suzhou Kintor Pharmaceuticals, Inc., Jiangsu 215123 (CN)
(72) Inventor: TONG, Youzhi, Suzhou, Jiangsu 215123 (CN); REN, Zhihua, Suzhou, Jiangsu 215123 (CN); YAN, Honghua, Suzhou, Jiangsu 215123 (CN); CAI, Xiang, Suzhou, Jiangsu 215123 (CN)
(74) Representative: Cabinet Becker et Associés
(86) International application number: PCT/CN2024/117422
(87) International publication number: WO 2025/051237

(57) **Abstract**

The present invention belongs to the technical field of medicine, and specifically relates to a pharmaceutical composition containing an androgen receptor inhibitor/antagonist, a method and the use. The use of the androgen receptor inhibitor/antagonist, in particular a combination of a compound of formula I or crystal form D of the compound of formula I and minoxidil, improves the effect obtained by using a single drug of the androgen receptor inhibitor/antagonist or minoxidil. In reality, such an improvement is synergistic in nature, because the effect of improving or promoting hair growth is also greater than that obtained by using the androgen receptor inhibitor/antagonist or minoxidil alone. The method is not only a simple physical combination, but also provides a good additive therapeutic effect, and has the advantage of good safety.

## Description

### Cross-Reference to Related Applications

The present disclosure claims the right of priority for the invention patent application filed in China on September 8, 2023, entitled "PHARMACEUTICAL COMPOSITION CONTAINING ANDROGEN RECEPTOR INHIBITOR/ANTAGONIST, METHOD AND USE" with the application number of CN 2023111578546, and the invention patent application filed in China on February 6, 2024, entitled "PHARMACEUTICAL COMPOSITION CONTAINING ANDROGEN RECEPTOR INHIBITOR/ANTAGONIST, METHOD AND USE" with the application number of CN 2024101716421, the entire contents of which are incorporated herein by reference.

### Technical Field

The present disclosure belongs to the technical field of medicine, and specifically relates to a pharmaceutical composition containing an androgen receptor inhibitor/antagonist and minoxidil as active ingredients, a method and the use.

### Background Art

Minoxidil is a potassium channel opener, and such drugs are often associated with reflex tachycardia and an increase in cardiac output when used for antihypertensive treatment. It can also reduce or stop hair loss and promote hair regrowth. Among the drugs approved in China for the treatment of alopecia and alopecia areata, minoxidil demonstrates good efficacy in promoting hair growth. The mechanisms of action of minoxidil are as follows: stimulating the proliferation of hair follicle epithelial cells and prolonging the anagen of hair growth; promoting angiogenesis and increasing local blood supply to provide nutrients for hair growth; and opening potassium ion channels, preventing the influx of calcium ions into cells, and increasing cellular DNA synthesis and hair follicle cell proliferation. Currently, various topical preparations of minoxidil have been available on the market both domestically and internationally, with high safety profiles. However, minoxidil acts mainly by improving microcirculatory disorders and accelerating the rate of hair growth, which constitutes a symptomatic treatment. Therefore, it demonstrates satisfactory efficacy only against common alopecia areata and alopecia, whereas its efficacy is unsatisfactory for more severe conditions such as alopecia totalis and alopecia universalis.

Androgenetic alopecia is an androgen-dependent hereditary disease and the most common clinical type of alopecia, characterized by a progressive reduction in hair density. Androgenetic alopecia in males is also known as male-pattern hair loss, while that in females is also known as female-pattern hair loss. Androgenetic alopecia is a polygenic disease inherited in an autosomal dominant manner. The local scalp hair follicles of patients exhibit increased sensitivity to androgens (mainly dihydrotestosterone), leading to follicle miniaturization and hair shaft thinning, with clinical manifestation of sparse and thinning hair. Chinese invention patent CN 102757389B discloses a small-molecule androgen receptor antagonist (the chemical name is 4-(4,4-dimethyl-3-(6-methylpyridin-3-yl)-5-oxo-2-thioxoimidazolidin-1-yl)-3-fluoro-2-methoxybenzonitrile (as shown in Formula I), which is currently in the clinical research phase).

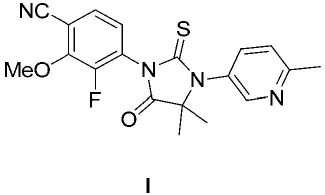

In the prior art, minoxidil is commonly used in combination with traditional Chinese medicine active ingredients that promote hair growth (e.g., Publication No. CN 109010432A, and Publication No. CN 113712968A), or in combination with the 5a reductase inhibitors finasteride (Publication No. CN 111973607A) and dutasteride (Publication No. CN 109674762A). Most such dual or multi-drug combinations involve merely simple physical mixing of individual agents that each exert hair growth-promoting effects as monotherapy, resulting in limited efficacy. Therefore, it is particularly important to develop a product and method for improving the monotherapeutic efficacy of minoxidil or androgen receptor inhibitors/antagonists.

### Summary of the Invention

### Problems to be solved by the disclosure

A primary object of the present disclosure is to provide a new pharmaceutical composition for a disease or condition associated with androgen receptor activity, and a treatment method and the use thereof.

### Solutions to solve problems

In a first aspect, the present disclosure provides a pharmaceutical composition comprising an androgen inhibitor/antagonist and minoxidil, or a pharmaceutically acceptable excipient thereof.

Further, the androgen inhibitor/antagonist comprises clascoterone, nilutamide, enzalutamide, topilutamide, bicalutamide, flutamide, hydrochlorothiazide, spironolactone, niclosamine, rezvilutamide, darolutamide, althiazide, apalutamide, deutenzalutamide, enobosarm, proxalutamide, ANA-001, 18F-dihydrotestosterone, 2-hydroxylflutamide, AKP-009, ASC-J9, AZD5312, EG017, LY2452473, MK-0773, VK5211, bavdegalutamide, dimethandrolone undecanoate, seviteronel, inocoterone, zanoterone, CP-COV03, FW-1022, APC-100, ARV-766, EM-5854, EPI-7386, MVI-118, ODM-204, TRC253, UT-34, ralaniten acetate, vosilasarm, FW-424, AC0176, AZD3514, BMS-641988, CB-03-10, CC-94676, DT-200, EZN-4176, GSK2849466, GSK971086, HP518, HRS-5041, HSK38008, MK-3984, ONC1-13B, PF-06260414, RO7656594, SXL01, TAS3681, TQB3720, TFF NIC, UNI911, FW-420, A031, AB001, AB006, AC-262536, ADA-308, AH-001, AR-V7 PROTAC, ARCC-4, ARD-2051, ARD-2128, ARD-2585, ARD-266, ARD-61, ARD-69, ASN-1780, ASR-600, BMS-564929, BWA-522, CA103, CB-03-04, CH4892280, CH5137291, CL-AR-100, CZ-212-3, DA-4210, EM 6537, HC-X022, HYG-410, HYG-420, HYG-430, HYG-440, ID119160021, IMB-A6, JMKX002992, JNJ-26146900, JNJ-28330835, JNJ-37654032, LG121071, LGD-2941, LGD-3303, LGD2226, LHJ-647, MTX-23, NUV-1156, NUV-1511, OLX104C, ONC2-13B, PARD-33, PF-0998425, PRX-304, RD162, RU 56187, RU 58642, RU 58841, RU 59063, SKLB-C2807, TD-802, VPC-13789, WS9761, XY-32, YM-175735, YM-92088, YM580, ZD3980, Zeta55, SCAI-502, XNTR-934, or XNTR-936.

Preferably, the present disclosure provides a pharmaceutical composition comprising an androgen inhibitor/antagonist and minoxidil, or a pharmaceutically acceptable excipient thereof, wherein the androgen inhibitor/antagonist is a compound of formula I or a pharmaceutically acceptable salt, stereoisomer, solvate, polymorph, isotopic derivative or prodrug thereof,

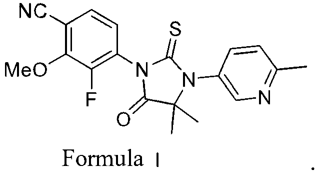

Further, in the present disclosure, the pharmaceutical composition comprises an androgen inhibitor/antagonist and minoxidil, or a pharmaceutically acceptable excipient thereof, wherein the androgen inhibitor/antagonist is an anhydrous polymorph of the compound of formula I.

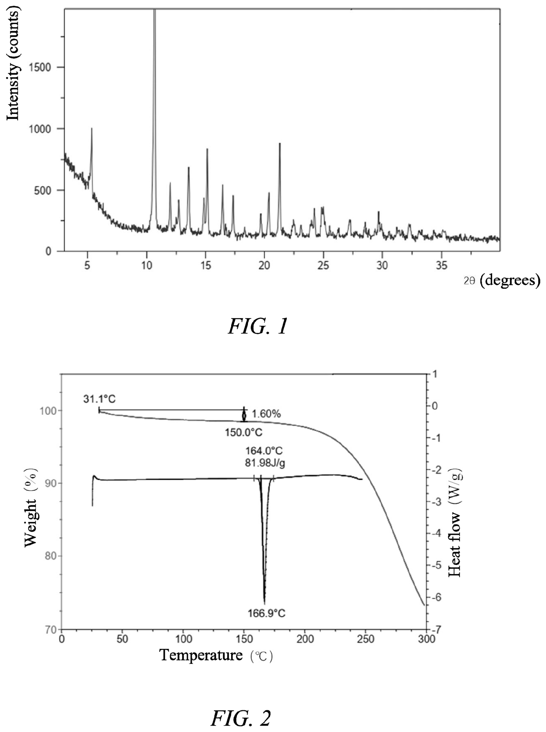

Still further, the anhydrous polymorph of the compound of formula I is crystal form D, wherein the crystal form D satisfies at least one of the following conditions:
I. the crystal form D has an XRPD pattern comprising peaks at 2θ values of: 5.3 ± 0.2°, 10.7 ± 0.2°, 13.5 ± 0.2°, 15.1 ± 0.2° and 21.3 ± 0.2°;
   preferably, the crystal form D has an XRPD pattern further comprising peaks at 2θ values of: 12.0 ± 0.2°, 12.7 ± 0.2°, 14.8 ± 0.2°, 16.4 ± 0.2°, 17.3 ± 0.2°, 20.3 ± 0.2°, 24.2 ± 0.2°and 24.8 ± 0.2°;
   more preferably, the crystal form D has an XRPD pattern further comprising peaks at 2θ values of: 19.7 ± 0.2°, 22.5 ± 0.2°, 23.1 ± 0.2°, 27.3 ± 0.2°, 28.5 ± 0.2°, 29.7 ± 0.2° and 32.3 ± 0.2°;
   further preferably, the crystal form D has an XRPD pattern substantially consistent with FIG. 1;
II. the crystal form D has a TGA pattern showing a weight loss of about 1.6% at 150 ± 1°C;
   preferably, the crystal form D has a TGA pattern substantially consistent with FIG. 2; and
III. the crystal form D has a DSC pattern showing an endotherm at 167 ± 1°C;
   preferably, the crystal form D has a DSC pattern substantially consistent with FIG. 2.

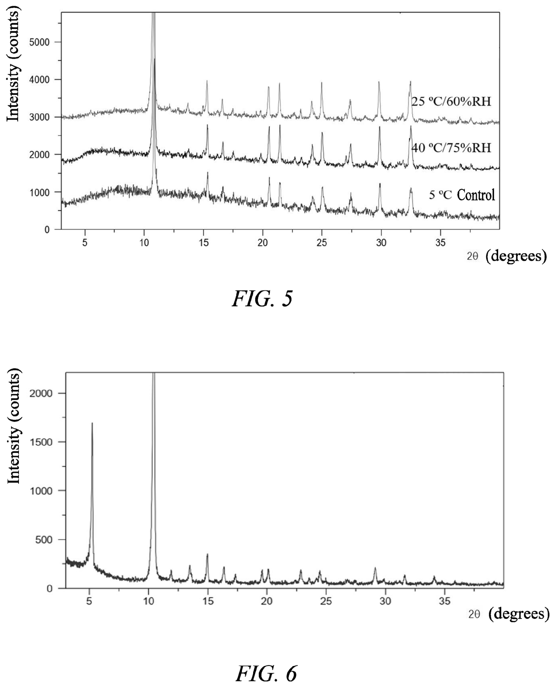

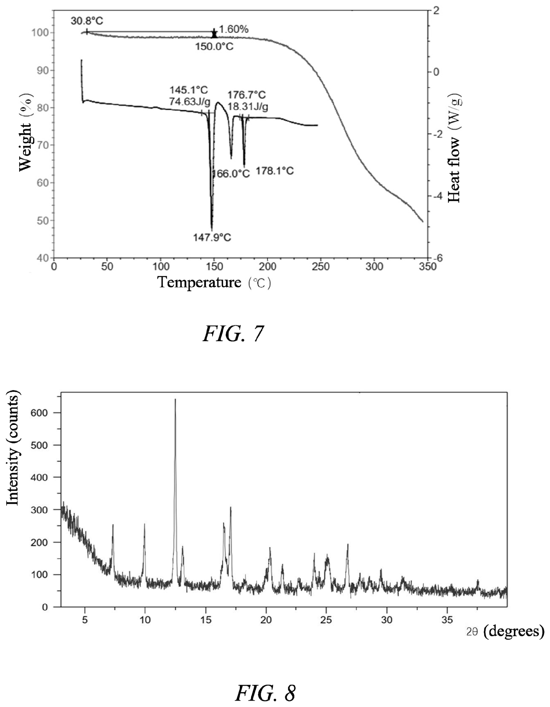

Still further, the anhydrous polymorph is crystal form C, and the crystal form C satisfies at least one of the following conditions:
I. the crystal form C has an XRPD pattern comprising peaks at 2θ values of: 5.2 ± 0.2°, 10.4 ± 0.2°, 13.4 ± 0.2°, 15.0 ± 0.2°, 16.4 ± 0.2° and 29.1 ± 0.2°;
   preferably, the crystal form C has an XRPD pattern further comprising peaks at 2θ values of: 19.6 ± 0.2°, 20.1 ± 0.2°, 22.8 ± 0.2° and 24.4 ± 0.2°;
   more preferably, the crystal form C has an XRPD pattern further comprising peaks at 2θ values of: 11.9 ± 0.2°, 17.3 ± 0.2°, 23.6 ± 0.2°, 31.6 ± 0.2° and 34.1 ± 0.2°;
   further preferably, the crystal form C has an XRPD pattern substantially consistent with FIG. 6;
II. the crystal form C has a TGA pattern showing a weight loss of about 1.6% at 150 ± 1°C;
   preferably, the crystal form C has a TGA pattern substantially consistent with FIG. 7; and
III. the crystal form C has a DSC pattern showing endotherms at 148 ± 1°C, 167 ± 1°C and 177 ± 1°C;
   preferably, the crystal form C has a DSC pattern substantially consistent with FIG. 7.

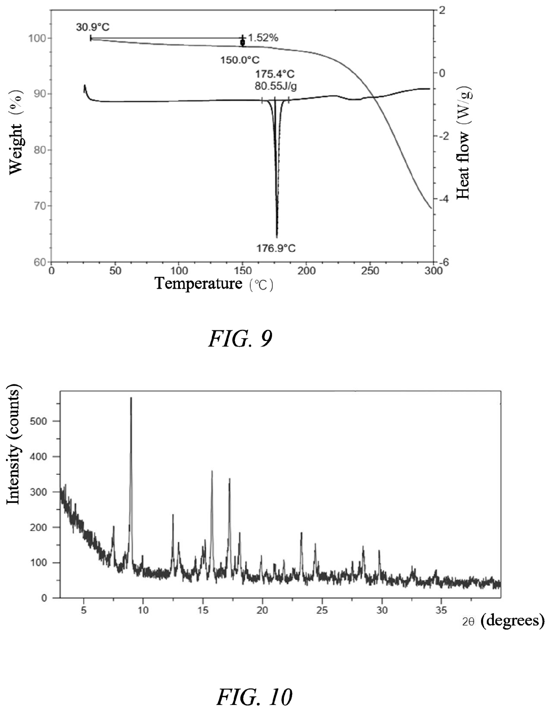

Still further, the anhydrous polymorph is crystal form B, and the crystal form B satisfies at least one of the following conditions:
I. the crystal form B has an XRPD pattern comprising peaks at 2θ values of: 7.3 ± 0.2°, 9.9 ± 0.2°, 12.5 ± 0.2°, 16.5 ± 0.2° and 17.1 ± 0.2°;
   preferably, the crystal form B has an XRPD pattern further comprising peaks at 2θ values of: 13.1 ± 0.2°, 20.3 ± 0.2°, 24.0 ± 0.2°, 25.2 ± 0.2° and 26.7 ± 0.2°;
   more preferably, the crystal form B has an XRPD pattern further comprising peaks at 2θ values of: 21.3 ± 0.2°, 27.8 ± 0.2°, 28.6 ± 0.2°, 29.5 ± 0.2° and 31.3 ± 0.2°;
   further preferably, the crystal form B has an XRPD pattern substantially consistent with FIG. 8;
II. the crystal form B has a TGA pattern showing a weight loss of about 1.5% at 150 ± 1°C;
   preferably, the crystal form B has a TGA pattern substantially consistent with FIG. 9; and
III. the crystal form B has a DSC pattern showing an endotherm at 177 ± 1°C;
   preferably, the crystal form B has a DSC pattern substantially consistent with FIG. 9.

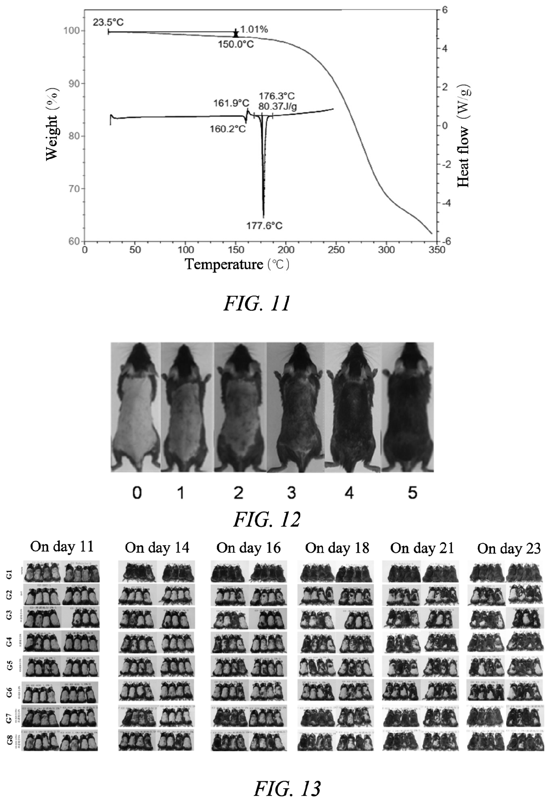

Still further, the anhydrous polymorph is crystal form A, and the crystal form A satisfies at least one of the following conditions:
the crystal form A has an XRPD pattern comprising peaks at 2θ values of: 9.0 ± 0.2°, 12.5 ± 0.2°, 15.7 ± 0.2°, 17.2 ± 0.2°, 18.1 ± 0.2° and 23.2 ± 0.2°;
preferably, the crystal form A has an XRPD pattern further comprising peaks at 2θ values of: 3.3 ± 0.2°, 7.5 ± 0.2°, 12.9 ± 0.2°, 15.2 ± 0.2°, 24.4 ± 0.2°, 28.4 ± 0.2° and 29.8 ± 0.2°;
more preferably, the crystal form A has an XRPD pattern further comprising peaks at 2θ values of: 14.4 ± 0.2°, 17.7 ± 0.2°, 19.9 ± 0.2° and 21.8 ± 0.2°;
further preferably, the crystal form A has an XRPD pattern substantially consistent with FIG. 10;
II. the crystal form A has a TGA pattern showing a weight loss of about 1.0% at 150 ± 1°C;
preferably, the crystal form A has a TGA pattern substantially consistent with FIG. 11; and
III. the crystal form A has a DSC pattern showing endotherms at 160 ± 1°C and 177 ± 1°C, and an exotherm at 162 ± 1°C;
preferably, the crystal form A has a DSC pattern substantially consistent with FIG. 11.

Further, in the present disclosure, the pharmaceutical composition is characterized in that
a therapeutically effective amount of the androgen inhibitor/antagonist is administered to an individual in a continuous daily regimen until progression of a disease or condition associated with androgen receptor activity or unacceptable toxicity occurs;
and/or, the androgen inhibitor/antagonist is administered in an amount of 0.05% (0.5 mg/mL or mg/g) to 10% (100 mg/mL or mg/g);
and/or, the androgen inhibitor/antagonist is administered in an amount of 0.25% (2.5 mg/mL or mg/g) to 1% (10 mg/mL or mg/g);
and/or, the androgen inhibitor/antagonist is administered in an amount of 0.1 mg/day-1000 mg/day;
and/or, the androgen inhibitor/antagonist is administered in an amount of 0.5 mg/day-100 mg/day;
and/or, the androgen inhibitor/antagonist is administered in an amount of 1 mg/day-50 mg/day;
and/or, the androgen inhibitor/antagonist is administered in an amount of 1.8 mg/day, 2.5 mg/day, 3.6 mg/day, 5 mg/day, 7.2 mg/day, 10 mg/day, or 20 mg/day;
and/or, the androgen inhibitor/antagonist is administered QD or BID;
and/or, the androgen inhibitor/antagonist is administered QD or BID in an amount of 0.25% (2.5 mg/mL or mg/g), 0.5% (5 mg/mL or mg/g) or 1% (10 mg/mL or mg/g).

Further, in the present disclosure, the pharmaceutical composition is characterized in that
a therapeutically effective amount of the minoxidil or the pharmaceutically acceptable excipient thereof is administered to an individual in a continuous daily regimen until progression of a disease or condition associated with androgen receptor activity or unacceptable toxicity occurs;
and/or, the minoxidil or the pharmaceutically acceptable excipient thereof is administered in an amount of 2 mg/day-500 mg/day;
and/or, the minoxidil or the pharmaceutically acceptable excipient thereof is administered in an amount of 14.4 mg/day, 20 mg/day, 36 mg/day, 40 mg/day, 44 mg/day, 50 mg/day, or 100 mg/day;
and/or, the minoxidil or the pharmaceutically acceptable excipient thereof is administered in an amount of 0.1% (1 mg/mL or mg/g) to 15% (150 mg/mL or mg/g);
and/or, the minoxidil or the pharmaceutically acceptable excipient thereof is administered in an amount of 2% (20 mg/mL or mg/g) to 5% (50 mg/mL or mg/g);
and/or, the minoxidil is administered QD or BID;
and/or, the minoxidil or the pharmaceutically acceptable excipient thereof is administered QD or BID in an amount of 2% (20 mg/mL or mg/g) or 5% (50 mg/mL or mg/g).

In a second aspect, the present disclosure provides the pharmaceutical composition of the first aspect, for use in the prevention, alleviation and/or treatment of a disease or condition associated with androgen receptor activity;
preferably, the disease or condition associated with androgen receptor activity is selected from prostate cancer, benign prostatic hyperplasia, acne, hypertrichosis, seborrhoea and androgenic alopecia.

In a third aspect, the present disclosure provides the use of the pharmaceutical composition of the first aspect in the preparation of a drug for preventing, alleviating and/or treating a disease or condition associated with androgen receptor activity;
preferably, the disease or condition associated with androgen receptor activity is selected from prostate cancer, benign prostatic hyperplasia, acne, hypertrichosis, seborrhoea and androgenic alopecia.

In a fourth aspect, the present disclosure provides a method for preventing, alleviating and/or treating a disease or condition associated with androgen receptor activity, comprising the following steps: administering a preventively, alleviatively and/or therapeutically effective amount of the pharmaceutical composition of the first aspect to an individual in need thereof;
preferably, the disease or condition associated with androgen receptor activity is selected from prostate cancer, benign prostatic hyperplasia, acne, hypertrichosis, seborrhoea and androgenic alopecia;
and/or, the androgen inhibitor/antagonist and minoxidil comprised in the pharmaceutical composition are co-formulated or separately formulated, and administered in combination, simultaneously or at intervals.

In a fifth aspect, the present disclosure provides a pharmaceutical composition for preventing, alleviating and/or treating androgenic alopecia, comprising a compound of formula I or crystal form D of the compound of formula I, and minoxidil or a pharmaceutically acceptable excipient thereof,

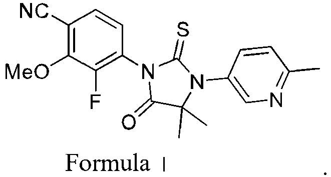

Preferably, the pharmaceutical composition is characterized in that
a therapeutically effective amount of an androgen inhibitor/antagonist (a compound of formula I or crystal form D of the compound of formula I) is administered to an individual in a continuous daily regimen until progression of a disease or condition associated with androgen receptor activity or unacceptable toxicity occurs;
and/or, the androgen inhibitor/antagonist (the compound of formula I or the crystal form D of the compound of formula I) is administered in an amount of 0.05% (0.5 mg/mL or mg/g) to 10% (100 mg/mL or mg/g);
and/or, the androgen inhibitor/antagonist (the compound of formula I or the crystal form D of the compound of formula I) is administered in an amount of 0.25% (2.5 mg/mL or mg/g) to 1% (10 mg/mL or mg/g);
and/or, the androgen inhibitor/antagonist (the compound of formula I or the crystal form D of the compound of formula I) is administered in an amount of 0.1 mg/day-1000 mg/day;
and/or, the androgen inhibitor/antagonist (the compound of formula I or the crystal form D of the compound of formula I) is administered in an amount of 0.5 mg/day-100 mg/day;
and/or, the androgen inhibitor/antagonist is administered in an amount of 1 mg/day-50 mg/day;
and/or, the androgen inhibitor/antagonist (the compound of formula I or the crystal form D of the compound of formula I) is administered in an amount of 1.8 mg/day, 2.5 mg/day, 3.6 mg/day, 5 mg/day, 7.2 mg/day, 10 mg/day, or 20 mg/day;
and/or, the androgen inhibitor/antagonist (the compound of formula I or the crystal form D of the compound of formula I) is administered QD or BID;
and/or, the androgen inhibitor/antagonist (the compound of formula I or the crystal form D of the compound of formula I) is administered QD or BID in an amount of 0.25% (2.5 mg/mL or mg/g), 0.5% (5 mg/mL or mg/g) or 1% (10 mg/mL or mg/g);
a therapeutically effective amount of the minoxidil or the pharmaceutically acceptable excipient thereof is administered to an individual in a continuous daily regimen until progression of a disease or condition associated with androgen receptor activity or unacceptable toxicity occurs;
and/or, the minoxidil or the pharmaceutically acceptable excipient thereof is administered in an amount of 2 mg/day-500 mg/day;
and/or, the minoxidil or the pharmaceutically acceptable excipient thereof is administered in an amount of 14.4 mg/day, 20 mg/day, 36 mg/day, 40 mg/day, 44 mg/day, 50 mg/day, or 100 mg/day;
and/or, the minoxidil or the pharmaceutically acceptable excipient thereof is administered in an amount of 0.1% (1 mg/mL or mg/g) to 15% (150 mg/mL or mg/g);
and/or, the minoxidil or the pharmaceutically acceptable excipient thereof is administered in an amount of 2% (20 mg/mL or mg/g) to 5% (50 mg/mL or mg/g);
and/or, the minoxidil is administered QD or BID;
and/or, the minoxidil or the pharmaceutically acceptable excipient thereof is administered QD or BID in an amount of 2% (20 mg/mL or mg/g) or 5% (50 mg/mL or mg/g).

More preferably, the pharmaceutical composition is characterized in that
the compound of formula I and minoxidil are administered via a combined administration mode selected from: simultaneous administration, separate formulation with co-administration, or separate formulation with sequential administration;
and/or, the administration route of the compound of formula I and minoxidil is selected from oral administration, parenteral administration, topical administration, and transdermal administration; preferably, the administration route is selected from topical administration and transdermal administration;
and/or, a therapeutically effective amount of the compound of formula I is administered to an individual in a continuous daily regimen until progression of an androgenic alopecia disease or condition or unacceptable toxicity occurs;
and/or, a preparation containing the compound of formula I is formulated at a strength of the compound of formula I from about 0.05% (0.5 mg/mL or mg/g) to 10% (100 mg/mL or mg/g) in the preparation; the preparation is a solid preparation, a liquid preparation (such as a topical solution), a foam, an aerosol, or a gel, preferably a liquid preparation (such as a topical solution). Preferably, a solution of the compound of formula I is formulated at a strength of the compound of formula I from about 0.25% (2.5 mg/mL or mg/g) to 5% (50 mg/mL or mg/g) in the solution;
and/or, the compound of formula I is administered in an amount of about 0.1 mg/day-about 1000 mg/day;
and/or, the compound of formula I is administered in an amount of about 0.5 mg/day-about 100 mg/day;
and/or, the compound of formula I is administered in an amount of about 1 mg/day-about 50 mg/day;
and/or, the compound of formula I is administered in an amount of about 1.8 mg/day, about 2.5 mg/day, about 3.6 mg/day, about 5 mg/day, about 7.2 mg/day, about 10 mg/day, or about 20 mg/day;
and/or, the compound of formula I is administered QD or BID;
and/or, a solution of the compound of formula I is formulated at a strength of the compound of formula I of about 0.25% (2.5 mg/mL), 0.5% (5 mg/mL) or 1% (10 mg/mL) in the solution and administered QD or BID via topical administration, with an administration volume of about 1 mL of the solution each time.

A therapeutically effective amount of the minoxidil is administered to an individual in a continuous daily regimen until progression of an androgenic alopecia disease or condition or unacceptable toxicity occurs;
and/or, the minoxidil is administered in an amount of about 2 mg/day-about 500 mg/day;
and/or, the minoxidil is administered in an amount of about 14.4 mg/day, about 20 mg/day, about 36 mg/day, about 40 mg/day, about 44 mg/day, about 50 mg/day, or about 100 mg/day;
and/or, a preparation containing the minoxidil is formulated at a strength of the minoxidil from about 0.1% (1 mg/mL or mg/g) to 15% (150 mg/mL or mg/g) in the preparation; the preparation is a solid preparation, a liquid preparation (such as a topical solution), a foam, an aerosol, or a gel, preferably a liquid preparation, a foam, and an aerosol;
and/or, a preparation containing the minoxidil is formulated and administered at a strength of the minoxidil from about 2% (20 mg/mL or mg/g) to 5% (50 mg/mL or mg/g) in the preparation; the preparation is a solid preparation, a liquid preparation (such as a topical solution), a foam, an aerosol, or a gel, preferably a liquid preparation, a foam, and an aerosol;
and/or, the minoxidil is administered QD or BID;
and/or, a solution, foam or aerosol containing the minoxidil is formulated and administered at a strength of the minoxidil from about 2% (20 mg/mL or mg/g) to 5% (50 mg/mL or mg/g) in the preparation;
and/or, a solution, foam or aerosol of the minoxidil is formulated at a strength of the minoxidil of about 2% (20 mg/mL or mg/g) or 5% (50 mg/mL or mg/g) in the preparation, and the solution is administered QD or BID via topical administration, with an administration volume of about 1 mL of the solution each time;
and/or, liquid preparations of the compound of formula I and the minoxidil are formulated separately and independently, and the preparation with a strength of the compound of formula I of about 0.25% (2.5 mg/mL), 0.5% (5 mg/mL) or 1% (10 mg/mL) is administered in combination with the preparation with a strength of the minoxidil of about 2% (20 mg/mL) or 5% (50 mg/mL); preferably, the compound of formula I and the minoxidil are administered QD or BID via topical administration; preferably, an administration volume is about 1 mL each time.

In some technical solutions of the present application, the pharmaceutical composition is characterized in that liquid preparations of the compound of formula I and the minoxidil are formulated separately and independently, with a strength of the compound of formula I of about 0.5% (5 mg/mL) and a strength of the minoxidil of about 2% (20 mg/mL); and/or, the compound of formula I and the minoxidil are administered QD; preferably, the compound of formula I and the minoxidil are administered simultaneously; and/or, an administration volume is about 1 mL each time, i.e., the compound of formula I is administered in an amount of about 5 mg/day, and the minoxidil is administered in an amount of 20 mg/day.

In some technical solutions of the present application, the pharmaceutical composition is characterized in that liquid preparations of the compound of formula I and the minoxidil are formulated separately and independently, with a strength of the compound of formula I of about 0.25% (2.5 mg/mL) and a strength of the minoxidil of about 5% (50 mg/mL); and/or, the compound of formula I and the minoxidil are administered QD; preferably, the compound of formula I and the minoxidil are administered simultaneously; and/or, an administration volume is about 1 mL each time, i.e., the compound of formula I is administered in an amount of about 2.5 mg/day, and the minoxidil is administered in an amount of 50 mg/day.

In some technical solutions of the present application, the pharmaceutical composition is characterized in that liquid preparations of the compound of formula I and the minoxidil are formulated separately and independently, with a strength of the compound of formula I of about 0.5% (5 mg/mL) and a strength of the minoxidil of about 5% (50 mg/mL); and/or, the compound of formula I and the minoxidil are administered QD; preferably, the compound of formula I and the minoxidil are administered simultaneously; and/or, an administration volume is about 1 mL each time, i.e., the compound of formula I is administered in an amount of about 5 mg/day, and the minoxidil is administered in an amount of 50 mg/day.

In some technical solutions of the present application, the pharmaceutical composition is characterized in that liquid preparations of the compound of formula I and the minoxidil are formulated separately and independently, with a strength of the compound of formula I of about 1% (10 mg/mL) and a strength of the minoxidil of about 5% (50 mg/mL); and/or, the compound of formula I and the minoxidil are administered QD; preferably, the compound of formula I and the minoxidil are administered simultaneously; and/or, an administration volume is about 1 mL each time, i.e., the compound of formula I is administered in an amount of about 10 mg/day, and the minoxidil is administered in an amount of 50 mg/day.

In some technical solutions of the present application, the pharmaceutical composition is characterized in that liquid preparations of the compound of formula I and the minoxidil are formulated separately and independently, with a strength of the compound of formula I of about 0.25% (2.5 mg/mL) and a strength of the minoxidil of about 5% (50 mg/mL); and/or, the compound of formula I and the minoxidil are administered BID; preferably, the compound of formula I and the minoxidil are administered at intervals; and/or, an administration volume is about 1 mL each time, i.e., the compound of formula I is administered in an amount of about 5 mg/day, and the minoxidil is administered in an amount of 100 mg/day.

In some technical solutions of the present application, the pharmaceutical composition is characterized in that liquid preparations of the compound of formula I and the minoxidil are formulated separately and independently, with a strength of the compound of formula I of about 0.5% (5 mg/mL) and a strength of the minoxidil of about 5% (50 mg/mL); and/or, the compound of formula I and the minoxidil are administered BID; preferably, the compound of formula I and the minoxidil are administered at intervals; and/or, an administration volume is about 1 mL each time, i.e., the compound of formula I is administered in an amount of about 10 mg/day, and the minoxidil is administered in an amount of 100 mg/day.

Preferably, in the above pharmaceutical composition, the weight percentage of the compound of formula I or the anhydrous polymorph (crystal form D) of the compound of formula I is 1.0%-99.0%, such as 10.0%-80.0%, 20%-80%, 25%-80%, 25%-70%, 25%-65%, 25%-60%, 25%-55%, 25%-50%, 30%-50%, 35%-50% or 40%-50%.

In a sixth aspect, the present disclosure provides a method for preventing, alleviating and/or treating androgenic alopecia, comprising the following steps: administering a preventively, alleviatively and/or therapeutically effective amount of a compound of formula I and minoxidil to an individual in need thereof;
and/or, in the method, the compound of formula I and minoxidil are administered via a combined administration mode selected from: simultaneous administration, separate formulation with co-administration, or separate formulation with sequential administration;
and/or, in the method, an administration route is selected from oral administration, parenteral administration, topical administration, and transdermal administration; preferably, in the method, the administration route is selected from topical administration and transdermal administration;
and/or, a therapeutically effective amount of the compound of formula I is administered to an individual in a continuous daily regimen until progression of an androgenic alopecia disease or condition or unacceptable toxicity occurs;
and/or, a preparation containing the compound of formula I is formulated and administered at a strength of the compound of formula I from about 0.05% (0.5 mg/mL or mg/g) to 10% (100 mg/mL or mg/g) in the preparation; the preparation is a solid preparation, a liquid preparation (such as a topical solution), a foam, an aerosol, or a gel, preferably a liquid preparation (such as a topical solution). Preferably, a solution of the compound of formula I is formulated and administered at a strength of the compound of formula I from about 0.25% (2.5 mg/mL or mg/g) to 5% (50 mg/mL or mg/g) in the solution;
and/or, the compound of formula I is administered in an amount of about 0.1 mg/day-about 1000 mg/day;
and/or, the compound of formula I is administered in an amount of about 0.5 mg/day-about 100 mg/day;
and/or, the compound of formula I is administered in an amount of about 1 mg/day-about 50 mg/day;
and/or, the compound of formula I is administered in an amount of about 1.8 mg/day, about 2.5 mg/day, about 3.6 mg/day, about 5 mg/day, about 7.2 mg/day, about 10 mg/day, or about 20 mg/day;
and/or, the compound of formula I is administered QD or BID;
and/or, a solution of the compound of formula I is formulated and administered QD or BID via topical administration at a strength of the compound of formula I of about 0.25% (2.5 mg/mL), 0.5% (5 mg/mL) or 1% (10 mg/mL) in the solution, with an administration volume of about 1 mL each time.

A therapeutically effective amount of the minoxidil is administered to an individual in a continuous daily regimen until progression of an androgenic alopecia disease or condition or unacceptable toxicity occurs;
and/or, the minoxidil is administered in an amount of about 2 mg/day-about 500 mg/day;
and/or, the minoxidil is administered in an amount of about 14.4 mg/day, about 20 mg/day, about 36 mg/day, about 40 mg/day, about 44 mg/day, about 50 mg/day, or about 100 mg/day;
and/or, a preparation containing the minoxidil is formulated and administered at a strength of the minoxidil from about 0.1% (1 mg/mL or mg/g) to 15% (150 mg/mL or mg/g) in the preparation; the preparation is a solid preparation, a liquid preparation (such as a topical solution), a foam, an aerosol, or a gel, preferably a liquid preparation, a foam, and an aerosol;
and/or, a preparation containing the minoxidil is formulated and administered at a strength of the minoxidil from about 2% (20 mg/mL or mg/g) to 5% (50 mg/mL or mg/g) in the preparation; the preparation is a solid preparation, a liquid preparation (such as a topical solution), a foam, an aerosol, or a gel, preferably a liquid preparation, a foam, and an aerosol;
and/or, the minoxidil is administered QD or BID;
and/or, a solution, foam or aerosol containing the minoxidil is formulated and administered at a strength of the minoxidil from about 2% (20 mg/mL or mg/g) to 5% (50 mg/mL or mg/g) in the preparation;
and/or, a solution, foam or aerosol of the minoxidil is formulated and administered QD or BID via topical administration at a strength of the minoxidil of about 2% (20 mg/mL or mg/g) or 5% (50 mg/mL or mg/g) in the preparation, with an administration volume of about 1 mL each time;
and/or, liquid preparations of the compound of formula I and the minoxidil are formulated separately and independently, and the preparation with a strength of the compound of formula I of about 0.25% (2.5 mg/mL), 0.5% (5 mg/mL) or 1% (10 mg/mL) is administered in combination with the preparation with a strength of the minoxidil of about 2% (20 mg/mL) or 5% (50 mg/mL); preferably, the compound of formula I and the minoxidil are administered QD or BID via topical administration; preferably, an administration volume is about 1 mL each time.

In some technical solutions of the present application, in the method: liquid preparations of the compound of formula I and the minoxidil are formulated separately and independently, with a strength of the compound of formula I of about 0.5% (5 mg/mL) and a strength of the minoxidil of about 2% (20 mg/mL); and/or, the compound of formula I and the minoxidil are administered QD; preferably, the compound of formula I and the minoxidil are administered simultaneously; and/or, an administration volume is about 1 mL each time, i.e., the compound of formula I is administered in an amount of about 5 mg/day, and the minoxidil is administered in an amount of 20 mg/day.

In some technical solutions of the present application, in the method: liquid preparations of the compound of formula I and the minoxidil are formulated separately and independently, with a strength of the compound of formula I of about 0.25% (2.5 mg/mL) and a strength of the minoxidil of about 5% (50 mg/mL); and/or, the compound of formula I and the minoxidil are administered QD; preferably, the compound of formula I and the minoxidil are administered simultaneously; and/or, an administration volume is about 1 mL each time, i.e., the compound of formula I is administered in an amount of about 2.5 mg/day, and the minoxidil is administered in an amount of 50 mg/day.

In some technical solutions of the present application, in the method: liquid preparations of the compound of formula I and the minoxidil are formulated separately and independently, with a strength of the compound of formula I of about 0.5% (5 mg/mL) and a strength of the minoxidil of about 5% (50 mg/mL); and/or, the compound of formula I and the minoxidil are administered QD; preferably, the compound of formula I and the minoxidil are administered simultaneously; and/or, an administration volume is about 1 mL each time, i.e., the compound of formula I is administered in an amount of about 5 mg/day, and the minoxidil is administered in an amount of 50 mg/day.

In some technical solutions of the present application, in the method: liquid preparations of the compound of formula I and the minoxidil are formulated separately and independently, with a strength of the compound of formula I of about 1% (10 mg/mL) and a strength of the minoxidil of about 5% (50 mg/mL); and/or, the compound of formula I and the minoxidil are administered QD; preferably, the compound of formula I and the minoxidil are administered simultaneously; and/or, an administration volume is about 1 mL each time, i.e., the compound of formula I is administered in an amount of about 10 mg/day, and the minoxidil is administered in an amount of 50 mg/day.

In some technical solutions of the present application, in the method: liquid preparations of the compound of formula I and the minoxidil are formulated separately and independently, with a strength of the compound of formula I of about 0.25% (2.5 mg/mL) and a strength of the minoxidil of about 5% (50 mg/mL); and/or, the compound of formula I and the minoxidil are administered BID; preferably, the compound of formula I and the minoxidil are administered at intervals; and/or, an administration volume is about 1 mL each time, i.e., the compound of formula I is administered in an amount of about 5 mg/day, and the minoxidil is administered in an amount of 100 mg/day.

In some technical solutions of the present application, in the method: liquid preparations of the compound of formula I and the minoxidil are formulated separately and independently, with a strength of the compound of formula I of about 0.5% (5 mg/mL) and a strength of the minoxidil of about 5% (50 mg/mL); and/or, the compound of formula I and the minoxidil are administered BID; preferably, the compound of formula I and the minoxidil are administered at intervals; and/or, an administration volume is about 1 mL each time, i.e., the compound of formula I is administered in an amount of about 10 mg/day, and the minoxidil is administered in an amount of 100 mg/day.

Preferably, in the method, the weight percentage of the compound of formula I is 1.0%-99.0%, such as 10.0%-80.0%, 20%-80%, 25%-80%, 25%-70%, 25%-65%, 25%-60%, 25%-55%, 25%-50%, 30%-50%, 35%-50% or 40%-50%.

### Effects of the disclosure

The present disclosure provides a pharmaceutical composition containing an androgen receptor inhibitor/antagonist and minoxidil as active ingredients, a method and the use. It has now been surprisingly found that the use of the androgen receptor inhibitor/antagonist, in particular a combination of a compound of formula I or crystal form D of the compound of formula I and minoxidil, improves the effect obtained by using a single drug of the androgen receptor inhibitor/antagonist or minoxidil. In reality, such an improvement is synergistic in nature, because the effect of improving or promoting hair growth is also greater than that obtained by using the androgen receptor inhibitor/antagonist or minoxidil alone. The method is not only a simple physical combination, but also provides a good additive therapeutic effect, and has the advantage of good safety.

### Brief Description of the Drawings

FIG. 1 shows an XRPD pattern of an anhydrous polymorph (crystal form D) of the compound of formula I;
FIG. 2 shows a TGA/DSC pattern of an anhydrous polymorph (crystal form D) of the compound of formula I;

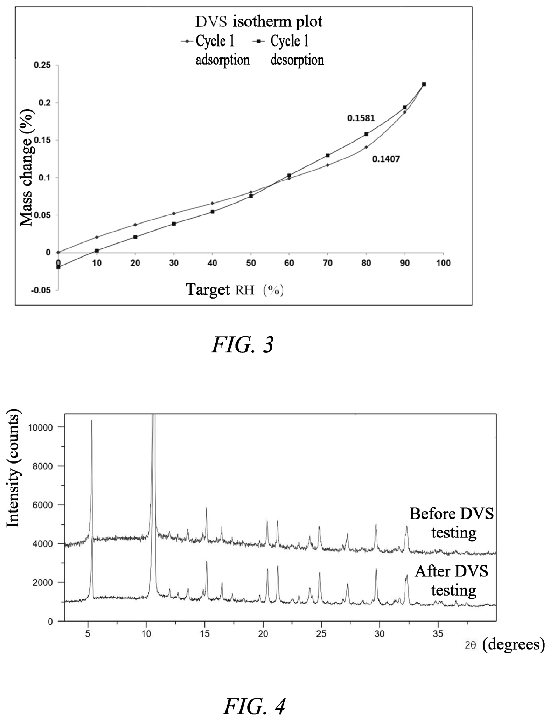

FIG. 3 shows a DVS pattern of an anhydrous polymorph (crystal form D) of the compound of formula I;
FIG. 4 shows an XRPD pattern of an anhydrous polymorph (crystal form D) of the compound of formula I before and after DVS testing;
FIG. 5 shows an XRPD pattern of an anhydrous polymorph (crystal form D) of the compound of formula I (stability testing);
FIG. 6 shows an XRPD pattern of an anhydrous polymorph (crystal form C) of the compound of formula I;
FIG. 7 shows a TGA/DSC pattern of an anhydrous polymorph (crystal form C) of the compound of formula I;
FIG. 8 shows an XRPD pattern of an anhydrous polymorph (crystal form B) of the compound of formula I;
FIG. 9 shows a TGA/DSC pattern of an anhydrous polymorph (crystal form B) of the compound of formula I;
FIG. 10 shows an XRPD pattern of an anhydrous polymorph (crystal form A) of the compound of formula I;
FIG. 11 shows a TGA/DSC pattern of an anhydrous polymorph (crystal form A) of the compound of formula I;
FIG. 12 shows a schematic diagram of main observation indicators in mice;
FIG. 13 shows a schematic diagram of the hair growth experiment in mice from groups G1-G8;
FIG. 14 shows a comparison chart of hair scoring in mice from groups G1-G8;
FIG. 15 shows a comparison chart of body weight results in mice from groups G1-G8;
FIG. 16 shows a schematic diagram of hair growth in mice from groups G1-G8;
FIG. 17 shows a comparison chart of hair scoring in mice from groups G1-G8; and
FIG. 18 shows a comparison chart of body weight results in mice from groups G1-G8.

### Detailed Description of Embodiments

Unless otherwise specified, all scientific and technical terms used herein have the meanings commonly understood by a person skilled in the art.

Unless otherwise specified, words in the singular form appearing herein, such as "a", "an" and "the", cover their corresponding plural referents, unless the context clearly indicates otherwise. For example, when referring to "a" crystal form or polymorph, different crystal form(s) or polymorph(s) are covered, and when referring to "the" method, equivalent steps and methods known to one of ordinary skill in the art are covered.

Unless otherwise specified, the term "comprise" and variations, such as "contain" and "include", appearing herein means that a set covers not only explicitly disclosed integer(s), step(s) or combinations thereof, but also does not exclude any other integers, steps or combinations thereof. Furthermore, in certain cases, the term "comprise" appearing herein can be replaced with the term "contain", "include" or "have".

Unless otherwise specified, the term "effective amount", "therapeutically effective amount" or "pharmaceutically effective amount" as used herein refers to an amount of a therapeutic agent sufficient to alleviate one or more aspects of the condition. This can result in the reduction and/or alleviation of signs, symptoms or etiology of the disease, or any other desired change in a biological system. For example, an "effective amount" for therapeutic uses is an amount of the composition provided herein required to achieve a clinically significant reduction in the prevention, alleviation and/or treatment of diseases or conditions associated with androgen receptor activity. For example, for a given aspect (e.g., incidence length), a therapeutically effective amount will exhibit an increase or decrease of 5%, 10%, 15%, 20%, 25%, 40%, 50%, 60%, 75%, 80%, 90% or at least 100%. The therapeutic effect can also be expressed as a "-fold" increase or decrease. For example, a therapeutically effective amount can produce an effect that is at least 1.2-fold, 1.5-fold, 2-fold, 5-fold or more, relative to a control. In any individual case, an appropriate "effective amount" can be determined by using techniques such as dose-escalation studies.

Unless otherwise specified, the pharmaceutical composition comprising the androgen inhibitor/antagonist of the present disclosure can be administered to an individual/subject/patient in need thereof via routes such as oral administration, inhalation administration, rectum administration, parenteral administration or topical administration, etc. For oral administration, the pharmaceutical composition can be in the form of a solid preparation (for example a tablet, a powder, a granule, a capsule, etc.) or a liquid preparation (for example a water-based or oil-based suspension or other liquid preparation, such as a syrup, a solution, etc.). For parenteral administration, the pharmaceutical composition can be in the form of a solution, a suspension, a freeze-dried powder, etc. The pharmaceutical composition can be a single unit having an accurate dose. In addition, the pharmaceutical composition can further comprise additional pharmacologically active ingredients.

Unless otherwise specified, the term "treatment" as used herein (and as is well known in the art) also generally includes any method for achieving a beneficial or desired outcome (including a clinical outcome) for a disorder in a subject. Beneficial or desired clinical outcomes include, but are not limited to, alleviation or alleviation of one or more symptoms or disorders, reduction in the severity of a disease, stabilisation (i.e., not worsening) of the disease state, prevention of disease spread or dissemination, delay or slowing of disease progression, improvement or alleviation of the disease state, reduction of the disease, and remission of symptoms, whether partial or complete and whether detectable or undetectable. In other words, the term "treatment" as used herein includes any cure, improvement, or prevention of a disease. Treatment can prevent the onset of a disease, inhibit the dissemination of a disease, alleviate the symptoms of a disease, completely or partially eliminate the underlying cause of the disease, shorten the duration of a disease, or a combination thereof.

Unless otherwise specified, the term "disease" refers to any deviation from the normal health in a mammal and includes a state in which symptoms of the disease are present, as well as a disorder in which a deviation (e.g., infection, gene mutation, genetic defect, etc.) has occurred but symptoms have not yet manifested. According to the present disclosure, the methods disclosed herein are suitable for use in a patient who is a member of the vertebrate class, including, but not limited to, primates, livestock, and domestic pets (e.g., companion animals). Typically, the patient is a human patient.

Unless otherwise specified, the term "topical application" or "topical administration" as used herein are used interchangeably and include the administration of the composition to the upper and/or lower eyelid margins, eyebrow area, scalp or face. Topical application or administration can result in the delivery of the active agent to a localized area of the skin or body.

Unless otherwise specified, the abbreviations used herein have their conventional meanings in the field of chemistry, biology or pharmacy.

Unless otherwise specified, the phrase "pharmaceutically acceptable salt" as used herein refers to a salt of an active compound that has the same pharmacological activity as the active compound and is biologically and otherwise advantageous.

Unless otherwise specified, the term "about" as used in the present disclosure indicates a tolerance of ± 5%. The terms QD and BID refer to administration frequencies of once daily and twice daily, respectively. It should be understood that the administration frequencies may be adjusted provided that the daily dosage is met, for example, once a day, twice a day, once every two days, or once every three days.

For the crystal forms herein, only the characteristic peaks (i.e., the most characteristic, significant, unique and/or repeatable diffraction peaks) in the XRPD pattern are summarized, while other peaks can be obtained from the pattern by conventional methods. The above-mentioned characteristic peaks can be reproducible within an error limit (error range: ± 0.2°).

In the present disclosure, when the androgen inhibitor/antagonist or minoxidil is described as being administered in an amount of 0.25% (2.5 mg/mL or mg/g) to 1% (10 mg/mL or mg/g), 1% means that 10 mg of the androgen inhibitor/antagonist or minoxidil is contained in 1 mL of the preparation (liquid preparation, such as a topical solution) or 1 g of the preparation (solid or semisolid preparation, such as a gel); the rest of the descriptions are analogous.

Androgenic alopecia is also known as androgenetic alopecia (AGA), which can be either male or female androgenetic alopecia. The present application is particularly directed to male androgenetic alopecia, such as a male patient with androgenetic alopecia of Hamilton-Norwood Grade IIIv-V.

### Source of minoxidil in embodiments of the present disclosure

Minoxidil (CAS 38304-91-5) is known for its efficacy in alleviating or preventing hair loss and promoting hair regrowth. It is available without a prescription for the treatment of androgenetic alopecia and other forms of alopecia; however, measurable changes disappear within months after discontinuation of treatment. The minoxidil preparations of the present disclosure are commercially available, for example, from Shanghai Macklin Biochemical Co., Ltd. Provided is minoxidil having a structure of 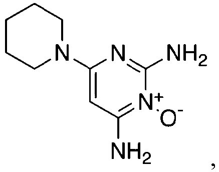 or a pharmaceutically acceptable excipient thereof.

Minoxidil at concentrations of 50 mg/mL (strength: 60 mL:3 g) and 20 mg/mL (strength: 60 mL:1.2 g) in the present disclosure were both purchased from Shanxi Zhendong Anxin Biopharmaceutical Co., Ltd.

### Source of dihydrotestosterone (DHT) in embodiments of the present disclosure

Dihydrotestosterone, a steroid hormone secreted by the testes, is a primary androgen in the human body and is related to the development of male secondary sexual characteristics. It is the primary androgen produced by the Leydig cells of the testes, and belongs to C10-steroids. After entering the circulating blood, the vast majority of dihydrotestosterone binds to sex hormone-binding globulin (SHBG) in the plasma, with a very small portion remaining in a free state. In the present disclosure, DHT is used to simulate androgen-mediated alopecia, and is commercially available, for example, from Suzhou Shengnuokang Biotechnology Co., Ltd.

### Source of compound of formula I in embodiments of the present disclosure

The title compound in a substantially gelatinous or viscous state was prepared accordance to the contents disclosed in paragraphs [0425] to [0427] on page 50 of the specification of CN 102757389B. The title compound was dissolved in DMSO, and then water was added dropwise to precipitate a solid. The filter cake was collected by filtration and dried under reduced pressure at 40-50°C to obtain a compound of formula I in a solid form.

The preparations of compounds of formula I with different concentrations can be prepared with reference to the technical teachings in CN 202310219411.9, the entire content of which is hereby incorporated into the present application by the applicant.

### Source of anhydrous polymorphs (crystal forms A, B, C and D) of compound of formula I in embodiments of the present disclosure

The anhydrous polymorphs (crystal forms A, B, C and D) were prepared according to the contents disclosed in Examples 1-4 of the specification of WO 2022199577A1.

In Example 1 of the present disclosure, the androgen inhibitor/antagonist is preferably a compound of formula I or crystal form D.

### Experimental materials and experimental animals of the present disclosure

Any solvent commonly used in the art can be used as a solvent for the pharmaceutical composition of the present disclosure, comprising an androgen inhibitor/antagonist and minoxidil for combined administration.

The present disclosure is further described by the following non-limiting examples.

### Experimental example 1: In-vivo efficacy experiment

### Experimental design

100 mice were deeply anesthetized. First, the hair on the back of the mice was shaved off with an animal hair clipper. Then, depilatory cream (Veet, https://item.jd.com/6291245.html) was evenly applied to the skin surface of the mice. After leaving it for 7-10 minutes, the remaining hair was gently scraped off against the hair growth direction with a depilatory spatula. Finally, the residual depilatory cream on the skin surface was thoroughly washed off with a wet wipe to avoid skin burns in the mice.

The main observation indicators of this experiment are shown in FIG. 12 (literature on hair scoring: Biomolecules & Therapeutics, 17(3), 241-248 (2009)), which is described as follows: (Score 0: No darkening of skin colour observed in any areas; Score 1: Darkening of skin area from greyish to black; Score 2: Visible short hairs; Score 3: Sparse hairs; Score 4: Dense hairs; Score 5: Complete hair growth). After depilation, the mice were photographed and scored three times a week until the end of the experiment.

### Experimental method

100 mice were deeply anesthetized. First, the hair on the back of the mice was shaved off with an animal hair clipper. Then, depilatory cream was evenly applied to the skin surface of the mice. After leaving it for 7-10 minutes, the remaining hair was gently scraped off against the hair growth direction with a depilatory spatula. Finally, the residual depilatory cream on the skin surface was thoroughly washed off with a wet wipe to avoid skin burns in the mice.

The experiment was divided into 8 groups in total, as shown in Table 1. DHT and test substances (compound of formula I and minoxidil) were administered starting on the day following depilation, which was recorded as Day 1 (the day of depilation was recorded as Day 0). 64 mice in good condition were randomly selected and assigned to each group.

**Table 1 Experimental grouping**

| Group | Number | Test substance | Administration dose | Test substance concentration | Administration volume | Administration route | Administration frequency & Administration period |
|---|---|---|---|---|---|---|---|
| G1 | 8 | Vehicle | Vehicle | - | 150 µL | Topical application | QD×23 |
| G2 | 8 | DHT | 30 mg/kg | 6 mg/mL | 100 µL/20 g | Subcutaneous injection | QD×23 |
| | | | Vehicle | - | 150 µL | Topical application | QD×23 |
| G3 | 8 | DHT | 30 mg/kg | 6 mg/mL | 100 µL/20 g | Subcutaneous injection | QD×23 |
| | | Minoxidil | 2% | 20 mg/mL | 150 µL | Topical application | QD×23 |
| G4 | 8 | DHT | 30 mg/kg | 6 mg/mL | 100 µL/20 g | Subcutaneous injection | QD×23 |
| | | Minoxidil | 5% | 50 mg/mL | 150 µL | Topical application | QD×23 |
| G5 | 8 | DHT | 30 mg/kg | 6 mg/mL | 100 µL/20 g | Subcutaneous injection | QD×23 |
| | | Compound of formula I | 0.5% | 5 mg/mL | 150 µL | Topical application | QD×23 |
| G6 | 8 | DHT | 30 mg/kg | 6 mg/mL | 100 µL/20 g | Subcutaneous injection | QD×23 |
| | | Compound of formula I | 1% | 10 mg/mL | 150 µL | Topical application | QD×23 |
| G7 | 8 | DHT | 30 mg/kg | 6 mg/mL | 100 µL/20 g | Subcutaneous injection | QD×23 |
| | | Compound of formula I | 0.5% | 5 mg/mL | 150 µL | Topical application | QD×23 |
| | | Minoxidil | 2% | 20 mg/mL | 150 µL | Topical application | QD×23 |
| G8 | 8 | DHT | 30 mg/kg | 6 mg/mL | 100 µL/20 g | Subcutaneous injection | QD×23 |
| | | Compound of formula I | 1% | 10 mg/mL | 150 µL | Topical application | QD×23 |
| | | Minoxidil | 5% | 50 mg/mL | 150 µL | Topical application | QD×23 |

The vehicle in Table 2 refers to a mixture of diethylene glycol monoethyl ether, dehydrated alcohol, propylene glycol and purified water.

According to the grouping in Table 1, and the corresponding administration dose, test substance concentration and administration volume, DHT, minoxidil and a compound of formula I were applied topically to the dorsal skin of mice treated according to the above experimental design and methods. For groups G7 and G8, which received a combined administration of the compound of formula I and minoxidil, one drug was administered first followed by the other, with the administration interval between the two drugs not exceeding 1 minute.

Starting from the first day of administration, body weight was weighed and recorded twice a week, and hair regrowth (anagen) scoring and photography were performed weekly, until the end of the experiment. The results on Day 23 are shown in FIG. 13. Scoring was conducted according to the criteria described in the experimental design.

### Experimental results

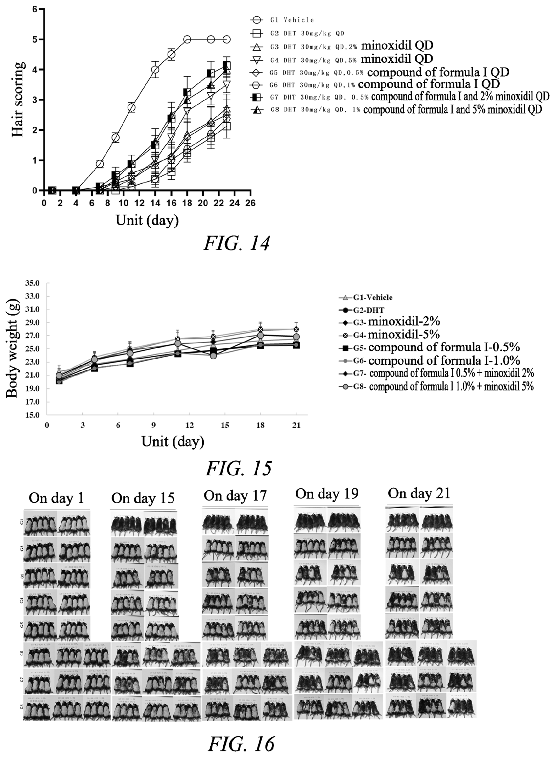

The results are shown in FIG. 14, Table 2 and Table 3. Corresponding to Table 1, G1 is the vehicle control group, G2 is the DHT model group, G3 is the 2% (20 mg/mL) minoxidil monotherapy group, G4 is the 5% (50 mg/mL) minoxidil monotherapy group, G5 is the 0.5% (5 mg/mL) compound of formula I monotherapy group, G6 is the 1.0% (10 mg/mL) compound of formula I monotherapy group, G7 is the combined administration group receiving 0.5% (5 mg/mL) compound of formula I and 2% (20 mg/mL) minoxidil, and G8 is the combined administration group receiving 1.0% (10 mg/mL) compound of formula I and 5% (50 mg/mL) minoxidil. The results demonstrated that the combined administration of minoxidil and the compound of formula I can promote hair growth. Compared with the monotherapy groups, the combined administration exhibited enhanced efficacy in promoting hair growth, indicating a synergistic effect.

As shown in FIG. 15, during the administration period, the test substances had no effect on the body weight of the mice, indicating good safety.

**Table 2 Hair scoring in mice**

| Score | Day 7 | Day 9 | Day 11 | Day 14 | Day 16 | Day 18 | Day 21 | Day 23 |
|---|---|---|---|---|---|---|---|---|
| G1-vehicle | 0.88 | 1.75 | 2.75 | 4.00 | 4.50 | 5.00 | 5.00 | 5.00 |
| G2-DHT | 0.00 | 0.00 | 0.13 | 0.38 | 0.63 | 1.25 | 1.75 | 2.13 |
| G3-minoxidil-2% | 0.00 | 0.29 | 0.57 | 0.86 | 1.14 | 1.86 | 2.29 | 2.71 |
| G4-minoxidil-5% | 0.13 | 0.25 | 0.38 | 1.00 | 1.75 | 2.63 | 3.13 | 3.50 |
| G5-compound of formula I -0.5% | 0.00 | 0.13 | 0.38 | 0.88 | 1.13 | 1.75 | 2.25 | 2.50 |
| G6-compound of formula I -1.0% | 0.00 | 0.13 | 0.13 | 0.38 | 1.00 | 1.38 | 1.88 | 2.38 |
| G7-compound of formula I 0.5%+ Minoxidil 2% | 0.13 | 0.50 | 0.88 | 1.50 | 2.38 | 3.25 | 3.88 | 4.13 |
| G8-compound of formula 11%+ Minoxidil 5% | 0.00 | 0.38 | 0.88 | 1.63 | 2.50 | 3.00 | 3.50 | 4.00 |

**Table 3 Statistical values of hair scoring in mice**

| Statistics | | p value | | | | | | | |
|---|---|---|---|---|---|---|---|---|---|
| | | Day 7 | Day 9 | Day 11 | Day 14 | Day 16 | Day 18 | Day 21 | Day 23 |
| Treatment groups vs. Model group | G3 VS G2 | NA | 0.172 | 0.203 | 0.276 | 0.33 | 0.315 | 0.381 | 0.355 |
| | G4 VS G2 | 0.351 | 0.17 | 0.281 | 0.074 | 0.016 | 0.014 | 0.011 | 0.012 |
| | G5 VS G2 | NA | 0.351 | 0.281 | 0.172 | 0.312 | 0.428 | 0.448 | 0.567 |
| | G6 VS G2 | NA | 0.351 | 1 | 1 | 0.429 | 0.786 | 0.809 | 0.655 |
| | G7 VS G2 | 0.351 | 0.104 | 0.043 | 0.01 | 0.002 | 0.006 | 0.002 | 0.001 |
| | G8 VS G2 | NA | 0.08 | 0.043 | 0.022 | 0.002 | 0.001 | 0.002 | 0.002 |
| Monotherap y groups vs. Combinatio n groups | G3 VS G7 | 0.351 | 0.533 | 0.483 | 0.234 | 0.056 | 0.081 | 0.023 | 0.022 |
| | G5 VS G7 | 0.351 | 0.233 | 0.172 | 0.178 | 0.038 | 0.063 | 0.027 | 0.014 |
| | G4 VS G8 | 0.351 | 0.619 | 0.172 | 0.23 | 0.176 | 0.429 | 0.362 | 0.207 |
| | G6 VS G8 | NA | 0.281 | 0.043 | 0.024 | 0.019 | 0.002 | 0.002 | 0.003 |

| | | | | | | | | | |
|---|---|---|---|---|---|---|---|---|---|
| **p* < 0.05, ***p* < 0.01*, ****p* < 0.001 | | | | | | | | | |

### Experimental example 2: In-vivo efficacy experiment

120 mice were deeply anesthetized. First, the hair on the back of the mice was shaved off with an animal hair clipper. Then, depilatory cream (Veet, https://item.jd.com/6291245.html) was evenly applied to the skin surface of the mice. After leaving it for 7-10 minutes, the remaining hair was gently scraped off against the hair growth direction with a depilatory spatula. Finally, the residual depilatory cream on the skin surface was thoroughly washed off with a wet wipe to avoid skin burns in the mice.

The experiment was divided into 8 groups in total, as shown in Table 4. DHT and test substances (compound of formula I and minoxidil) were administered starting on the day following depilation, which was recorded as Day 1 (the day of depilation was recorded as Day 0). 86 mice in good condition were randomly selected and assigned to each group.

The main observation indicators of this experiment are shown in FIG. 12 (literature on hair scoring: Biomolecules & Therapeutics, 17(3), 241-248 (2009)), which is described as follows: (Score 0: No darkening of skin colour observed in any areas; Score 1: Darkening of skin area from greyish to black; Score 2: Visible short hairs; Score 3: Sparse hairs; Score 4: Dense hairs; Score 5: Complete hair growth). After depilation, the mice were photographed and scored three times a week until the end of the experiment.

**Table 4 Experimental grouping**

| Group | Number | Test substance | Administration dose | Test substance concentration | Administration volume | Administration route | Administration frequency & Administration period |
|---|---|---|---|---|---|---|---|
| G1 | 10 | Vehicle | Vehicle | - | 150 µL | Topical application | QD×21 |
| G2 | 10 | DHT | 30 mg/kg | 6 mg/mL | 100 µL/20 g | Subcutaneous injection | QD×21 |
| | | | Vehicle | - | 150 µL | Topical application | QD×21 |
| G3 | 10 | DHT | 30 mg/kg | 6 mg/mL | 100 µL/20 g | Subcutaneous injection | QD×21 |
| | | Minoxidil | 5% | 50 mg/mL | 150 µL | Topical application | QD×21 |
| G4 | 10 | DHT | 30 mg/kg | 6 mg/mL | 100 µL/20 g | Subcutaneous injection | QD×21 |
| | | Compound of formula I | 0.25% | 2.5 mg/mL | 150 µL | Topical application | QD×21 |
| G5 | 10 | DHT | 30 mg/kg | 6 mg/ml | 100 µL/20 g | Subcutaneous injection | QD×21 |
| | | Compound of formula I | 0.5% | 5 mg/mlL | 150 µL | Topical application | QD×21 |
| G6 | 12 | DHT | 30 mg/kg | 6 mg/mL | 100 µL/20 g | Subcutaneous injection | QD×21 |
| | | Compound of formula I | 0.25% | 2.5 mg/mL | 150 µL | Topical application | QD×21 |
| | | Minoxidil | 5% | 50 mg/mL | 150 µL | Topical application | QD×21 |
| G7 | 12 | DHT | 30 mg/kg | 6 mg/mL | 100 µL/20 g | Subcutaneous injection | QD×21 |
| | | Compound of formula I | 0.5% | 5 mg/mL | 150 µL | Topical application | QD×21 |
| | | Minoxidil | 5% | 50 mg/mL | 150 µL | Topical application | QD×21 |
| G8 | 12 | DHT | 30 mg/kg | 6 mg/mL | 100 µL/20 g | Subcutaneous injection | QD×21 |
| | | Compound of formula I | 1% | 10 mg/mL | 150 µL | Topical application | QD×21 |
| | | Minoxidil | 5% | 50 mg/mL | 150 µL | Topical application | QD×21 |

The vehicle in Table 4 refers to a mixture of diethylene glycol monoethyl ether, dehydrated alcohol, propylene glycol and purified water.

According to the grouping in Table 4, and the corresponding administration dose, test substance concentration and administration volume, DHT, minoxidil and a compound of formula I were applied topically to the dorsal skin of mice treated according to the above experimental design and methods. For groups G6, G7 and G8, which received a combined administration of the compound of formula I and minoxidil, one drug was administered first followed by the other, with the administration interval between the two drugs not exceeding 1 minute.

Starting from the first day of administration, body weight was weighed and recorded twice a week, and hair regrowth (anagen) scoring and photography were performed weekly, until the end of the experiment. The results on Day 21 are shown in FIG. 16. Scoring was conducted according to the criteria described in the experimental design.

### Experimental results

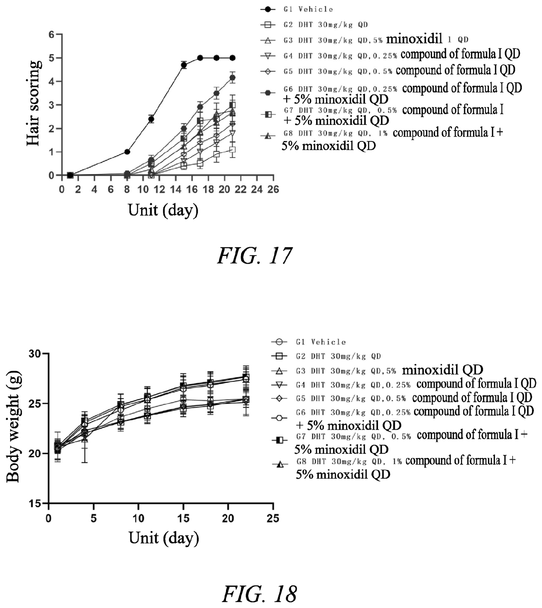

The results are shown in FIG. 17, Table 5 and Table 6. Corresponding to Table 4, G1 is the vehicle control group, G2 is the DHT model group, G3 is the 5% (50 mg/ml) minoxidil monotherapy group, G4 is the 0.25% (2.5 mg/ml) compound of formula I monotherapy group, G5 is the 0.5% (5 mg/ml) compound of formula I monotherapy group, G6 is the combined administration group receiving 0.25% (2.5 mg/ml) compound of formula I and 5% (50 mg/ml) minoxidil, G7 is the combined administration group receiving 0.5% (5 mg/ml) compound of formula I and 5% (50 mg/ml) minoxidil, and G8 is the combined administration group receiving 1.0% (10 mg/ml) compound of formula I and 5% (50 mg/ml) minoxidil. The results demonstrated that the combined administration of minoxidil and the compound of formula I can promote hair growth. Compared with the monotherapy groups, the combined administration exhibited enhanced efficacy in promoting hair growth, indicating a synergistic effect.

As shown in FIG. 18, during the administration period, the test substances had no effect on the body weight of the mice, indicating good safety.

**Table 5 Hair scoring in mice**

| Group | Day 8 | Day 11 | Day 15 | Day 17 | Day 19 | Day 21 |
|---|---|---|---|---|---|---|
| G1-vehicle | 1.00 | 2.40 | 4.70 | 5.00 | 5.00 | 5.00 |
| G2-DHT | 0.00 | 0.00 | 0.40 | 0.50 | 0.90 | 1.10 |
| G3-minoxidil-5% | 0.00 | 0.25 | 1.25 | 1.88 | 2.63 | 2.75 |
| G4-0.25% compound of formula I | 0.00 | 0.00 | 0.60 | 1.00 | 1.40 | 1.80 |
| G5-0.5% compound of formula I | 0.00 | 0.00 | 0.90 | 1.40 | 1.70 | 2.20 |
| G6 : 0.25% compound of formula I + 5% Minoxidil | 0.08 | 0.67 | 2.00 | 2.92 | 3.50 | 4.17 |
| G7 : 0.5% compound of formula I + 5% Minoxidil | 0.00 | 0.50 | 1.58 | 2.33 | 2.42 | 3.00 |
| G8 : 1% compound of formula I + 5% Minoxidil | 0.00 | 0.25 | 1.25 | 1.83 | 2.25 | 2.67 |

**Table 6 Statistical values of hair scoring in mice**

| Statistics | | p value | | | | | |
|---|---|---|---|---|---|---|---|
| | | Day 8 | Day 11 | Day 15 | Day 17 | Day 19 | Day 21 |
| Treatment groups vs. Model group | G3 VS G2 | NA | 0.170 | 0.035 | 0.009 | 0.008 | 0.010 |
| | G4 VS G2 | NA | NA | 0.476 | 0.229 | 0.339 | 0.196 |
| | G5 VS G2 | NA | NA | 0.096 | 0.040 | 0.099 | 0.033 |
| | G6 VS G2 | 0.374 | 0.005 | 0.000 | 0.000 | 0.000 | 0.000 |
| | G7 VS G2 | NA | NA | 0.003 | 0.001 | 0.011 | 0.003 |
| | G8 VS G2 | NA | 0.082 | 0.021 | 0.001 | 0.014 | 0.015 |
| Monotherapy groups vs. Combination groups | G6 VS G3 | 0.429 | 0.136 | 0.055 | 0.034 | 0.092 | 0.008 |
| | G6 VS G4 | 0.374 | 0.005 | 0.000 | 0.000 | 0.000 | 0.000 |
| | G7 VS G3 | NA | 0.288 | 0.454 | 0.443 | 0.739 | 0.701 |
| | G7 VS G5 | NA | NA | 0.088 | 0.086 | 0.180 | 0.165 |
| | G8 VS G3 | NA | 1.000 | 1.000 | 0.933 | 0.519 | 0.902 |
| **p* < **0.05,** ***p* < 0.01*, ****p* < 0.001 | | | | | | | |

Experimental Example 3: Clinical Trial on the Efficacy of KX-826 Combined with Minoxidil for the Treatment of AGA
I. General Information
   (i) Research Subjects 95 Chinese adult male patients with androgenetic alopecia (AGA) were screened and randomly divided into a combination group (48 cases) and a single-drug group (47 cases) at a 1:1 ratio.
   (ii) Inclusion Criteria
      - Male, aged 18-49 years, voluntarily signed the informed consent form;
      - Met the diagnostic criteria of the 2019 version of the *Guidelines for Diagnosis and Treatment of Androgenetic Alopecia in Chinese*, with a Hamilton-Norwood scale of IIIv-V, and voluntarily accepted tattooing of the target area;
      - Maintained a fixed hairstyle, hair color, and hair length during the follow-up period; had no plan for procreation within the study period and 3 months after the last administration, and adopted high-efficiency contraceptive measures.
   (iii) Exclusion Criteria
      1. Combined with alopecia diseases other than AGA, systemic diseases affecting hair growth, or scalp diseases;
      2. Presence of white hair in the target hair loss area, or a history of hair transplantation/hair extensions, or long-term wearing of wigs;
      3. Used 5α-reductase inhibitors, oral/topical minoxidil within 12 months before screening, or used anti-androgen drugs, immunosuppressants, or other drugs affecting efficacy within 6 months;
      4. History of hypotension/poorly controlled hypertension, HIV/HBV/HCV infection, history of malignant tumors (except cured carcinoma in situ), or allergy to trial drug ingredients;
      5. Participated in other interventional clinical trials within 3 months before screening, or underwent major surgery within 6 months.
II. Research Methods
   (i) Grouping and Dosing Regimen
      - Combination Group: 0.5% KX-826 tincture + 5% Minoxidil tincture, used topically twice daily (BID); 1 mL of each (approximately 7 sprays) per application. Apply one drug first and massage for 3-5 minutes for absorption, wait for an interval of ≥5 minutes before applying the other; use when hair/scalp is completely dry. The interval between morning and evening doses is ≥8 hours, and the total daily dose is ≤2 mL.
      - Single-drug 5% Minoxidil Group: 5% Minoxidil tincture, used topically BID; usage and dosage are the same as the minoxidil usage specifications in the combination group.
      - Trial Cycle: Screening period (maximum 2 weeks) + Treatment period (24 weeks) + Safety follow-up period (14 days after the last administration).
   (ii) Evaluation Index Change in Target Area Non-Vellus Hair Count (TAHC) at week 24 compared to baseline (unit: hairs/cm²).
   (iii) Statistical Analysis SAS 9.4 software was used for analysis. Quantitative data are described as mean ± standard deviation; the significance level for efficacy index testing is α=0.1.
III. Research Results
   1.Baseline TAHC of subjects in both groups: Combination group 133.52±37.08 vs. single-drug group 134.49±32.277 hairs/cm²; balanced and comparable, with no significant difference.
   2.Efficacy Index (TAHC change): At week 24, TAHC in the combination group increased by 31.51 hairs/cm² compared to baseline, and the single-drug group increased by 20.47 hairs/cm². The combination group was significantly superior to the single-drug group.

Experimental Example 4: Clinical Efficacy Trial of 0.5% KX-826 for AGA Treatment This trial is a multicenter, randomized, double-blind, placebo-controlled Phase III clinical trial (KX0826-CN-1003) to evaluate KX-826 tincture in Chinese adult male patients with androgenetic alopecia (AGA). A total of 740 adult male AGA subjects (Hamilton-Norwood scale IIIv, IV, V) were enrolled and randomly assigned to the 0.5% KX-826 tincture BID group and the placebo group at a 1:1 ratio. Treatment lasted 24 weeks according to the prescribed dosage, during which subjects received regular efficacy and safety inspections and evaluations. The subject screening criteria, evaluation indicators, and analysis methods of this experimental example are basically the same as those of Experimental Example 3.

Summary of Main Results: The mean baseline TAHC of subjects in this study was 110.83±30.651 hairs/cm².

Main Efficacy Results: In the 0.5% KX-826 BID group, TAHC in the target area increased by 8.33 hairs/cm² at week 24 compared to baseline, showing a statistical difference (p < 0.0001). Compared with the placebo group, KX-826 tincture showed a numerical advantage in TAHC increase, but the difference did not reach statistical significance.

Overall clinical trial data shows that 0.5% KX-826 combined with 5% Minoxidil is significantly superior to 5% Minoxidil tincture monotherapy in the primary efficacy endpoint (change in TAHC at week 24 from baseline), demonstrating a synergistic effect.

Although the embodiments of the present disclosure have been shown and described above, it should be understood that the above-described embodiments are merely exemplary and should not be construed as limiting the present disclosure. A person skilled in the art may make changes, modifications, replacements and variations to the above-described embodiments within the scope of the present disclosure, all of which should fall within the scope of protection of the present disclosure.

## Claims

1. A pharmaceutical composition comprising an androgen inhibitor/antagonist and minoxidil, or a pharmaceutically acceptable excipient thereof.

2. The pharmaceutical composition according to claim 1, **characterized in that** the androgen inhibitor/antagonist comprises clascoterone, nilutamide, enzalutamide, topilutamide, bicalutamide, flutamide, hydrochlorothiazide, spironolactone, niclosamine, rezvilutamide, darolutamide, althiazide, apalutamide, deutenzalutamide, enobosarm, proxalutamide, ANA-001, 18F-dihydrotestosterone, 2-hydroxylflutamide, AKP-009, ASC-J9, AZD5312, EG017, LY2452473, MK-0773, VK5211, bavdegalutamide, dimethandrolone undecanoate, seviteronel, inocoterone, zanoterone, CP-COV03, FW-1022, APC-100, ARV-766, EM-5854, EPI-7386, MVI-118, ODM-204, TRC253, UT-34, ralaniten acetate, vosilasarm, FW-424, AC0176, AZD3514, BMS-641988, CB-03-10, CC-94676, DT-200, EZN-4176, GSK2849466, GSK971086, HP518, HRS-5041, HSK38008, MK-3984, ONC1-13B, PF-06260414, RO7656594, SXL01, TAS3681, TQB3720, TFF NIC, UNI911, FW-420, A031, AB001, AB006, AC-262536, ADA-308, AH-001, AR-V7 PROTAC, ARCC-4, ARD-2051, ARD-2128, ARD-2585, ARD-266, ARD-61, ARD-69, ASN-1780, ASR-600, BMS-564929, BWA-522, CA103, CB-03-04, CH4892280, CH5137291, CL-AR-100, CZ-212-3, DA-4210, EM 6537, HC-X022, HYG-410, HYG-420, HYG-430, HYG-440, ID119160021, IMB-A6, JMKX002992, JNJ-26146900, JNJ-28330835, JNJ-37654032, LG121071, LGD-2941, LGD-3303, LGD2226, LHJ-647, MTX-23, NUV-1156, NUV-1511, OLX104C, ONC2-13B, PARD-33, PF-0998425, PRX-304, RD162, RU 56187, RU 58642, RU 58841, RU 59063, SKLB-C2807, TD-802, VPC-13789, WS9761, XY-32, YM-175735, YM-92088, YM580, ZD3980, Zeta55, SCAI-502, XNTR-934, or XNTR-936.

3. A pharmaceutical composition comprising an androgen inhibitor/antagonist and minoxidil, or a pharmaceutically acceptable excipient thereof, **characterized in that** the androgen inhibitor/antagonist is a compound of formula I or a pharmaceutically acceptable salt, stereoisomer, solvate, polymorph, isotopic derivative or prodrug thereof, 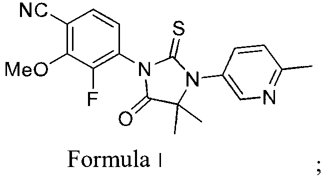 preferably, the androgen inhibitor/antagonist is an anhydrous polymorph of the compound of formula I.

4. The pharmaceutical composition according to claim 3, **characterized in that** the anhydrous polymorph of the compound of formula I is crystal form D, and the crystal form D satisfies at least one of the following conditions:
I. the crystal form D has an XRPD pattern comprising peaks at 2θ values of: 5.3 ± 0.2°, 10.7 ± 0.2°, 13.5 ± 0.2°, 15.1 ± 0.2° and 21.3 ± 0.2°;
preferably, the crystal form D has an XRPD pattern further comprising peaks at 2θ values of: 12.0 ± 0.2°, 12.7 ± 0.2°, 14.8 ± 0.2°, 16.4 ± 0.2°, 17.3 ± 0.2°, 20.3 ± 0.2°, 24.2 ± 0.2°and 24.8 ± 0.2°;
more preferably, the crystal form D has an XRPD pattern further comprising peaks at 2θ values of: 19.7 ± 0.2°, 22.5 ± 0.2°, 23.1 ± 0.2°, 27.3 ± 0.2°, 28.5 ± 0.2°, 29.7 ± 0.2° and 32.3 ± 0.2°;
further preferably, the crystal form D has an XRPD pattern substantially consistent with FIG. 1;
II. the crystal form D has a TGA pattern showing a weight loss of about 1.6% at 150 ± 1°C;
preferably, the crystal form D has a TGA pattern substantially consistent with FIG. 2; and
III. the crystal form D has a DSC pattern showing an endotherm at 167 ± 1°C;
preferably, the crystal form D has a DSC pattern substantially consistent with FIG. 2;
or the anhydrous polymorph is crystal form C, and the crystal form C satisfies at least one of the following conditions:
I. the crystal form C has an XRPD pattern comprising peaks at 2θ values of: 5.2 ± 0.2°, 10.4 ± 0.2°, 13.4 ± 0.2°, 15.0 ± 0.2°, 16.4 ± 0.2° and 29.1 ± 0.2°;
preferably, the crystal form C has an XRPD pattern further comprising peaks at 2θ values of: 19.6 ± 0.2°, 20.1 ± 0.2°, 22.8 ± 0.2° and 24.4 ± 0.2°;
more preferably, the crystal form C has an XRPD pattern further comprising peaks at 2θ values of: 11.9 ± 0.2°, 17.3 ± 0.2°, 23.6 ± 0.2°, 31.6 ± 0.2° and 34.1 ± 0.2°;
further preferably, the crystal form C has an XRPD pattern substantially consistent with FIG. 6;
II. the crystal form C has a TGA pattern showing a weight loss of about 1.6% at 150 ± 1°C;
preferably, the crystal form C has a TGA pattern substantially consistent with FIG. 7; and
III. the crystal form C has a DSC pattern showing endotherms at 148 ± 1°C, 167 ± 1°C and 177 ± 1°C;
preferably, the crystal form C has a DSC pattern substantially consistent with FIG. 7;
or the anhydrous polymorph is crystal form B, and the crystal form B satisfies at least one of the following conditions:
I. the crystal form B has an XRPD pattern comprising peaks at 2θ values of: 7.3 ± 0.2°, 9.9 ± 0.2°, 12.5 ± 0.2°, 16.5 ± 0.2° and 17.1 ± 0.2°;
preferably, the crystal form B has an XRPD pattern further comprising peaks at 2θ values of: 13.1 ± 0.2°, 20.3 ± 0.2°, 24.0 ± 0.2°, 25.2 ± 0.2° and 26.7 = 0.2°;
more preferably, the crystal form B has an XRPD pattern further comprising peaks at 2θ values of: 21.3 ± 0.2°, 27.8 ± 0.2°, 28.6 ± 0.2°, 29.5 ± 0.2° and 31.3 ± 0.2°;
further preferably, the crystal form B has an XRPD pattern substantially consistent with FIG. 8;
II. the crystal form B has a TGA pattern showing a weight loss of about 1.5% at 150 ± 1°C;
preferably, the crystal form B has a TGA pattern substantially consistent with FIG. 9; and
III. the crystal form B has a DSC pattern showing an endotherm at 177 ± 1°C;
preferably, the crystal form B has a DSC pattern substantially consistent with FIG. 9;
or the anhydrous polymorph is crystal form A, and the crystal form A satisfies at least one of the following conditions:
the crystal form A has an XRPD pattern comprising peaks at 2θ values of: 9.0 ± 0.2°, 12.5 ± 0.2°, 15.7 ± 0.2°, 17.2 ± 0.2°, 18.1 ± 0.2° and 23.2 ± 0.2°;
preferably, the crystal form A has an XRPD pattern further comprising peaks at 2θ values of: 3.3 ± 0.2°, 7.5 ± 0.2°, 12.9 ± 0.2°, 15.2 ± 0.2°, 24.4 ± 0.2°, 28.4 ± 0.2° and 29.8 ± 0.2°;
more preferably, the crystal form A has an XRPD pattern further comprising peaks at 2θ values of: 14.4 ± 0.2°, 17.7 ± 0.2°, 19.9 ± 0.2° and 21.8 ± 0.2°;
further preferably, the crystal form A has an XRPD pattern substantially consistent with FIG. 10;
II. the crystal form A has a TGA pattern showing a weight loss of about 1.0% at 150 ± 1°C;
preferably, the crystal form A has a TGA pattern substantially consistent with FIG. 11; and
III. the crystal form A has a DSC pattern showing endotherms at 160 ± 1°C and 177 ± 1°C, and an exotherm at 162 ± 1°C;
preferably, the crystal form A has a DSC pattern substantially consistent with FIG. 11.

5. The pharmaceutical composition according to any one of claims 1 to 4, **characterized in that**
a therapeutically effective amount of the androgen inhibitor/antagonist is administered to an individual in a continuous daily regimen until progression of a disease or condition associated with androgen receptor activity or unacceptable toxicity occurs;
and/or, the individual is a male or female patient with androgenetic alopecia, preferably a male patient with androgenetic alopecia, and further preferably a male patient with androgenetic alopecia of Hamilton-Norwood Grade IIIv-V;
and/or, the androgen inhibitor/antagonist is administered in an amount of 0.05% (0.5 mg/mL or mg/g) to 10% (100 mg/mL or mg/g);
and/or, the androgen inhibitor/antagonist is administered in an amount of 0.25% (2.5 mg/mL or mg/g) to 1% (10 mg/mL or mg/g);
and/or, the androgen inhibitor/antagonist is administered in an amount of 0.1 mg/day-1000 mg/day;
and/or, the androgen inhibitor/antagonist is administered in an amount of 0.5 mg/day-100 mg/day;
and/or, the androgen inhibitor/antagonist is administered in an amount of 1.8 mg/day, 2.5 mg/day, 1.8 mg/day, 3.6 mg/day, 5 mg/day, 10 mg/day, or 20 mg/day;
and/or, the androgen inhibitor/antagonist is administered QD or BID;
and/or, the androgen inhibitor/antagonist is administered QD or BID in an amount of 0.25% (2.5 mg/mL or mg/g), 0.5% (5 mg/mL or mg/g) or 1% (10 mg/mL or mg/g).

6. The pharmaceutical composition according to any one of claims 1 to 5, **characterized in that**
a therapeutically effective amount of the minoxidil or the pharmaceutically acceptable excipient thereof is administered to an individual in a continuous daily regimen until progression of a disease or condition associated with androgen receptor activity or unacceptable toxicity occurs;
and/or, the minoxidil or the pharmaceutically acceptable excipient thereof is administered in an amount of 2 mg/day-500 mg/day;
and/or, the minoxidil or the pharmaceutically acceptable excipient thereof is administered in an amount of 14.4 mg/day, 20 mg/day, 36 mg/day, 40 mg/day, 44 mg/day, 50 mg/day, or 100 mg/day;
and/or, the minoxidil or the pharmaceutically acceptable excipient thereof is administered in an amount of 0.1% (1 mg/mL or mg/g) to 15% (150 mg/mL or mg/g);
and/or, the minoxidil or the pharmaceutically acceptable excipient thereof is administered in an amount of 2% (20 mg/mL or mg/g) to 5% (50 mg/mL or mg/g);
and/or, the minoxidil is administered QD or BID;
and/or, the minoxidil or the pharmaceutically acceptable excipient thereof is administered QD or BID in an amount of 2% (20 mg/mL or mg/g) or 5% (50 mg/mL or mg/g).

7. The pharmaceutical composition according to any one of claims 1 to 6, **characterized in that** the pharmaceutical composition is used in the prevention, alleviation and/or treatment of a disease or condition associated with androgen receptor activity;
preferably, the disease or condition associated with androgen receptor activity is selected from prostate cancer, benign prostatic hyperplasia, acne, hypertrichosis, seborrhoea and androgenic alopecia;
and/or, a patient with the associated disease or condition is a male or female patient with androgenetic alopecia, preferably a male patient with androgenetic alopecia, and further preferably a male patient with androgenetic alopecia of Hamilton-Norwood Grade IIIv-V.

8. Use of the pharmaceutical composition according to any one of claims 1 to 6 in the preparation of a drug for preventing, alleviating and/or treating a disease or condition associated with androgen receptor activity, **characterized in that**
preferably, the disease or condition associated with androgen receptor activity is selected from prostate cancer, benign prostatic hyperplasia, acne, hypertrichosis, seborrhoea and androgenic alopecia;
and/or, a patient with the associated disease or condition is a male or female patient with androgenetic alopecia, preferably a male patient with androgenetic alopecia, and further preferably a male patient with androgenetic alopecia of Hamilton-Norwood Grade IIIv-V.

9. A method for preventing, alleviating and/or treating a disease or condition associated with androgen receptor activity, **characterized in that** the method comprises the following steps: administering a preventively, alleviatively and/or therapeutically effective amount of the pharmaceutical composition according to any one of claims 1 to 6 to an individual in need thereof;
preferably, the disease or condition associated with androgen receptor activity is selected from prostate cancer, benign prostatic hyperplasia, acne, hypertrichosis, seborrhoea and androgenic alopecia;
and/or, the androgen inhibitor/antagonist and minoxidil comprised in the pharmaceutical composition are co-formulated or separately formulated, and administered in combination, simultaneously or at intervals;
and/or, the pharmaceutical composition is topically administered to the individual in need thereof;
and/or, the individual is a male or female patient with androgenetic alopecia, preferably a male patient with androgenetic alopecia, and further preferably a male patient with androgenetic alopecia of Hamilton-Norwood Grade IIIv-V.

10. A pharmaceutical composition for preventing, alleviating and/or treating androgenic alopecia, **characterized in that** the pharmaceutical composition comprises a compound of formula I or crystal form D of the compound of formula I, and minoxidil or a pharmaceutically acceptable excipient thereof,

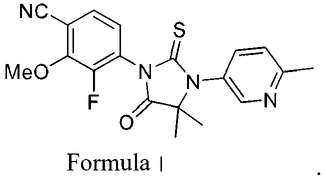

11. The pharmaceutical composition according to claim 10, **characterized in that**
a therapeutically effective amount of the compound of formula I or the crystal form D of the compound of formula I is administered to an individual in a continuous daily regimen until progression of a disease or condition associated with androgen receptor activity or unacceptable toxicity occurs;
and/or, the individual is a male or female patient with androgenetic alopecia, preferably a male patient with androgenetic alopecia, and further preferably a male patient with androgenetic alopecia of Hamilton-Norwood Grade IIIv-V;
and/or, the androgen inhibitor/antagonist is administered in an amount of 0.05% (0.5 mg/mL or mg/g) to 10% (100 mg/mL or mg/g);
and/or, the androgen inhibitor/antagonist is administered in an amount of 0.25% (2.5 mg/mL or mg/g) to 1% (10 mg/mL or mg/g);
and/or, the androgen inhibitor/antagonist is administered in an amount of 0.1 mg/day-1000 mg/day;
and/or, the androgen inhibitor/antagonist is administered in an amount of 0.5 mg/day-100 mg/day;
and/or, the androgen inhibitor/antagonist is administered in an amount of 1 mg/day-50 mg/day;
and/or, the androgen inhibitor/antagonist is administered in an amount of 2.5 mg/day, 5 mg/day, 10 mg/day, or 20 mg/day;
and/or, the androgen inhibitor/antagonist is administered QD or BID;
and/or, the androgen inhibitor/antagonist is administered QD or BID in an amount of 0.25% (2.5 mg/mL or mg/g), 0.5% (5 mg/mL or mg/g) or 1% (10 mg/mL or mg/g);
a therapeutically effective amount of the minoxidil or the pharmaceutically acceptable excipient thereof is administered to an individual in a continuous daily regimen until progression of a disease or condition associated with androgen receptor activity or unacceptable toxicity occurs;
and/or, the minoxidil or the pharmaceutically acceptable excipient thereof is administered in an amount of 2 mg/day-500 mg/day;
and/or, the minoxidil or the pharmaceutically acceptable excipient thereof is administered in an amount of 20 mg/day, 40 mg/day, 44 mg/day, 50 mg/day, or 100 mg/day;
and/or, the minoxidil or the pharmaceutically acceptable excipient thereof is administered in an amount of 0.1% (1 mg/mL or mg/g) to 15% (150 mg/mL or mg/g);
and/or, the minoxidil or the pharmaceutically acceptable excipient thereof is administered in an amount of 2% (20 mg/mL or mg/g) to 5% (50 mg/mL or mg/g);
and/or, the androgen inhibitor/antagonist is administered QD or BID;
and/or, the minoxidil or the pharmaceutically acceptable excipient thereof is administered QD or BID in an amount of 2% (20 mg/mL or mg/g) or 5% (50 mg/mL or mg/g).

12. The pharmaceutical composition according to claim 11, **characterized in that**
(1) the compound of formula I or the crystal form D of the compound of formula I is administered in an amount of 0.5% (5 mg/mL or mg/g), and the minoxidil is administered in an amount of 2% (20 mg/mL or mg/g); and/or, the compound of formula I or the crystal form D of the compound of formula I is administered in an amount of 5 mg/day; and/or, the minoxidil is administered in an amount of 20 mg/day; and/or, the compound of formula I or the crystal form D of the compound of formula I and minoxidil are administered QD; and/or, the compound of formula I or the crystal form D of the compound of formula I and minoxidil are administered simultaneously;
or (2) the compound of formula I or the crystal form D of the compound of formula I is administered in an amount of 0.25% (2.5 mg/mL or mg/g), and the minoxidil is administered in an amount of 5% (50 mg/mL or mg/g); and/or, the compound of formula I or the crystal form D of the compound of formula I is administered in an amount of 2.5 mg/day; and/or, the minoxidil is administered in an amount of 50 mg/day; and/or, the compound of formula I or the crystal form D of the compound of formula I and minoxidil are administered QD; and/or, the compound of formula I or the crystal form D of the compound of formula I and minoxidil are administered simultaneously;
or (3) the compound of formula I or the crystal form D of the compound of formula I is administered in an amount of 0.5% (5 mg/mL or mg/g), and the minoxidil is administered in an amount of 5% (50 mg/mL or mg/g); and/or, the compound of formula I or the crystal form D of the compound of formula I is administered in an amount of 5 mg/day; and/or, the minoxidil is administered in an amount of 50 mg/day; and/or, the compound of formula I or the crystal form D of the compound of formula I and minoxidil are administered QD; and/or, the compound of formula I or the crystal form D of the compound of formula I and minoxidil are administered simultaneously;
or (4) the compound of formula I or the crystal form D of the compound of formula I is administered in an amount of 1% (10 mg/mL or mg/g), and the minoxidil is administered in an amount of 5% (50 mg/mL or mg/g); and/or, the compound of formula I or the crystal form D of the compound of formula I is administered in an amount of 10 mg/day; and/or, the minoxidil is administered in an amount of 50 mg/day; and/or, the compound of formula I or the crystal form D of the compound of formula I and minoxidil are administered QD; and/or, the compound of formula I or the crystal form D of the compound of formula I and minoxidil are administered simultaneously;
or (5) the compound of formula I or the crystal form D of the compound of formula I is administered in an amount of 0.25% (2.5 mg/mL or mg/g), and the minoxidil is administered in an amount of 5% (50 mg/mL or mg/g); and/or, the compound of formula I or the crystal form D of the compound of formula I is administered in an amount of 5 mg/day; and/or, the minoxidil is administered in an amount of 100 mg/day; and/or, the compound of formula I or the crystal form D of the compound of formula I and minoxidil are administered BID; and/or, the compound of formula I or the crystal form D of the compound of formula I and minoxidil are administered at intervals;
or (6) the compound of formula I or the crystal form D of the compound of formula I is administered in an amount of 0.5% (5 mg/mL or mg/g), and the minoxidil is administered in an amount of 5% (50 mg/mL or mg/g); and/or, the compound of formula I or the crystal form D of the compound of formula I is administered in an amount of 10 mg/day; and/or, the minoxidil is administered in an amount of 100 mg/day; and/or, the compound of formula I or the crystal form D of the compound of formula I and minoxidil are administered BID; and/or, the compound of formula I or the crystal form D of the compound of formula I and minoxidil are administered at intervals.

13. A method for preventing, alleviating and/or treating androgenic alopecia, **characterized in that** the method comprises the following steps: administering a preventively, alleviatively and/or therapeutically effective amount of a compound of formula I and minoxidil to an individual in need thereof;
and/or, the individual is a male or female patient with androgenetic alopecia, preferably a male patient with androgenetic alopecia, and further preferably a male patient with androgenetic alopecia of Hamilton-Norwood Grade IIIv-V;
and/or, in the method, the compound of formula I and minoxidil are administered via a combined administration mode selected from: simultaneous administration, separate formulation with co-administration, or separate formulation with sequential administration;
and/or, in the method, an administration route is selected from oral administration, parenteral administration, topical administration, and transdermal administration; preferably, in the method, the administration route is selected from topical administration and transdermal administration;
and/or, a therapeutically effective amount of the compound of formula I is administered to an individual in a continuous daily regimen until progression of an androgenic alopecia disease or condition or unacceptable toxicity occurs;
and/or, a preparation containing the compound of formula I is formulated and administered at a strength of the compound of formula I from about 0.05% (0.5 mg/mL or mg/g) to 10% (100 mg/mL or mg/g) in the preparation; the preparation is a solid preparation, a liquid preparation (such as a topical solution), a foam, an aerosol, or a gel, preferably a liquid preparation (such as a topical solution); preferably, a solution of the compound of formula I is formulated and administered at a strength of the compound of formula I from about 0.25% (2.5 mg/mL or mg/g) to 5% (50 mg/mL or mg/g) in the solution;
and/or, the compound of formula I is administered in an amount of about 0.1 mg/day-about 1000 mg/day;
and/or, the compound of formula I is administered in an amount of about 0.5 mg/day-about 100 mg/day;
and/or, the compound of formula I is administered in an amount of about 1 mg/day-about 50 mg/day;
and/or, the compound of formula I is administered in an amount of about 1.8 mg/day, about 2.5 mg/day, about 3.6 mg/day, about 5 mg/day, about 7.2 mg/day, about 10 mg/day, or about 20 mg/day;
and/or, the compound of formula I is administered QD or BID;
and/or, a solution of the compound of formula I is formulated and administered QD or BID via topical administration at a strength of the compound of formula I of about 0.25% (2.5 mg/mL), 0.5% (5 mg/mL) or 1% (10 mg/mL) in the solution, with an administration volume of about 1 mL each time;
a therapeutically effective amount of the minoxidil is administered to an individual in a continuous daily regimen until progression of an androgenic alopecia disease or condition or unacceptable toxicity occurs;
and/or, the minoxidil is administered in an amount of about 2 mg/day-about 500 mg/day; and/or, the minoxidil is administered in an amount of about 14.4 mg/day, about 20 mg/day, about 36 mg/day, about 40 mg/day, about 44 mg/day, about 50 mg/day, or about 100 mg/day;
and/or, a preparation containing the minoxidil is formulated and administered at a strength of the minoxidil from about 0.1% (1 mg/mL or mg/g) to 15% (150 mg/mL or mg/g) in the preparation; the preparation is a solid preparation, a liquid preparation (such as a topical solution), a foam, an aerosol, or a gel, preferably a liquid preparation, a foam, and an aerosol;
and/or, a preparation containing the minoxidil is formulated and administered at a strength of the minoxidil from about 2% (20 mg/mL or mg/g) to 5% (50 mg/mL or mg/g) in the preparation; the preparation is a solid preparation, a liquid preparation (such as a topical solution), a foam, an aerosol, or a gel, preferably a liquid preparation, a foam, and an aerosol;
and/or, the minoxidil is administered QD or BID;
and/or, a solution, foam or aerosol containing the minoxidil is formulated and administered at a strength of the minoxidil from about 2% (20 mg/mL or mg/g) to 5% (50 mg/mL or mg/g) in the preparation;
and/or, a solution, foam or aerosol of the minoxidil is formulated and administered QD or BID via topical administration at a strength of the minoxidil of about 2% (20 mg/mL or mg/g) or 5% (50 mg/mL or mg/g) in the preparation, with an administration volume of about 1 mL each time;
and/or, liquid preparations of the compound of formula I and the minoxidil are formulated separately and independently, and the preparation with a strength of the compound of formula I of about 0.25% (2.5 mg/mL), 0.5% (5 mg/mL) or 1% (10 mg/mL) is administered in combination with the preparation with a strength of the minoxidil of about 2% (20 mg/mL) or 5% (50 mg/mL); preferably, the compound of formula I and the minoxidil are administered QD or BID via topical administration; preferably, an administration volume is about 1 mL each time.

14. The method according to claim 13, **characterized in that**
(1) in the method: liquid preparations of the compound of formula I and the minoxidil are formulated separately and independently, with a strength of the compound of formula I of about 0.5% (5 mg/mL) and a strength of the minoxidil of about 2% (20 mg/mL); and/or, the compound of formula I and the minoxidil are administered QD; and/or, an administration volume is about 1 mL each time, i.e., the compound of formula I is administered in an amount of about 5 mg/day, and the minoxidil is administered in an amount of 20 mg/day;
or (2) in the method: liquid preparations of the compound of formula I and the minoxidil are formulated separately and independently, with a strength of the compound of formula I of about 0.25% (2.5 mg/mL) and a strength of the minoxidil of about 5% (50 mg/mL); and/or, the compound of formula I and the minoxidil are administered QD; and/or, an administration volume is about 1 mL each time, i.e., the compound of formula I is administered in an amount of about 2.5 mg/day, and the minoxidil is administered in an amount of 50 mg/day;
or (3) in the method: liquid preparations of the compound of formula I and the minoxidil are formulated separately and independently, with a strength of the compound of formula I of about 0.5% (5 mg/mL) and a strength of the minoxidil of about 5% (50 mg/mL); and/or, the compound of formula I and the minoxidil are administered QD; and/or, an administration volume is about 1 mL each time, i.e., the compound of formula I is administered in an amount of about 5 mg/day, and the minoxidil is administered in an amount of 50 mg/day;
or (4) in the method: liquid preparations of the compound of formula I and the minoxidil are formulated separately and independently, with a strength of the compound of formula I of about 1% (10 mg/mL) and a strength of the minoxidil of about 5% (50 mg/mL); and/or, the compound of formula I and the minoxidil are administered QD; preferably, the compound of formula I and the minoxidil are administered simultaneously; and/or, an administration volume is about 1 mL each time, i.e., the compound of formula I is administered in an amount of about 10 mg/day, and the minoxidil is administered in an amount of 50 mg/day;
or (5) in the method: liquid preparations of the compound of formula I and the minoxidil are formulated separately and independently, with a strength of the compound of formula I of about 0.25% (2.5 mg/mL) and a strength of the minoxidil of about 5% (50 mg/mL); and/or, the compound of formula I and the minoxidil are administered BID; preferably, the compound of formula I and the minoxidil are administered at intervals; and/or, an administration volume is about 1 mL each time, i.e., the compound of formula I is administered in an amount of about 5 mg/day, and the minoxidil is administered in an amount of 100 mg/day;
or (6) in the method: liquid preparations of the compound of formula I and the minoxidil are formulated separately and independently, with a strength of the compound of formula I of about 0.5% (5 mg/mL) and a strength of the minoxidil of about 5% (50 mg/mL); and/or, the compound of formula I and the minoxidil are administered BID; preferably, the compound of formula I and the minoxidil are administered at intervals; and/or, an administration volume is about 1 mL each time, i.e., the compound of formula I is administered in an amount of about 10 mg/day, and the minoxidil is administered in an amount of 100 mg/day.
